# EUROPEAN PATENT APPLICATION

(11) **EP 2 526 990 A2**
(43) Date of publication of application: **28.11.2012**
(21) Application number: 12162702.0
(22) Date of filing: 12.10.2006
(51) Int. Cl.: A61M 15/00, A61K 9/00

(54) **A dry powder for inhalation**

(30) Priority: 12.10.2005 GB 0520794
(62) Divisional of application: 06808339.3
(71) Applicant: Innovata Biomed Limited, Chippenham Wiltshire SN14 6FH (GB)
(72) Inventor: Martyn, Glen Patrick, Chippenham, Wiltshire SN14 6FH (GB)
(74) Representative: Probert, Gareth David

(57) **Abstract**

A dry powder for inhalation comprising a desiccant and a bioactive agent.

## Description

The field of the invention is dry powder inhalers and compositions for use therein. This invention relates to an inhaler, in particular to an inhaler that may be disposable and suitable for single use, to the use of such an inhaler in methods of treatment, and to the compositions for use in inhalers of the invention.

### BACKGROUND OF THE INVENTION

Inhalers are used to deliver drugs into a patient's lungs. Typically, an inhaler contains or provides a mixture of drug particles and air or propellant gas. The mixture is delivered via the patient inhaling from a mouthpiece on the inhaler with the air or propellant gas carrying the drug particles into the patient's lungs.

In dry powder inhalers, the drug particles, in the form of a fine dry powder, are entrained into an airflow, and inhaled by the patient, for treatment for various conditions, for example, bronchial asthma. Drugs delivered via a dry powder inhaler can be used to treat many conditions, including those unrelated to lung conditions, via the systemic absorption of the drug into the bloodstream, via the lung.

Inhalation can also provide very rapid onset of the effect of the medicine. Inhalation may also allow smaller doses to be used to achieve the same desired results as orally ingested medicines.

For effective dose delivery using a dry powder inhaler, the powder particles must first be dispersed to form a powder/air aerosol. Various techniques for forming powder aerosols have been proposed. Some of these techniques use the airflow from the patient's inspiration alone to disperse the powder. Other techniques involve forming a powder aerosol by spinning a propeller within a chamber; generating a fast moving flow of air over or through the powder; and shaking, vibrating, or impacting a powder laden string, tape, or mesh, using mechanical devices or ultrasonics. In addition, various other techniques for generating powder aerosols have been proposed or used, with varying degrees of success. Challenges remain in achieving a dry powder inhaler which can effectively create a dry powder aerosol for inhalation, while also having advantages in other areas, such as effectiveness in creating an aerosol, reliability, complexity of design, costs, ergonomics, dose consistency, accountability to the environment, control of moisture ingress, resistance to moisture, and development of an inhalation device suited to only the occasional requirement for the active medicament, etc.

Metered dose inhalers and multiple dose dry powder inhalers are not intended or well designed for one-time use, to deliver a single dose. These types of inhalers are typically too bulky, costly, inefficient, or difficult to use, when only a single dose is desired, and where the inhaler and its drug/dose unit can be practically discarded after use, in an environmentally acceptable way. While certain drugs, such as asthma drugs, may be taken several times daily, other drugs, including certain peptides or proteins, are typically taken less frequently.

Due to the delay in using these types of drugs after they are removed from their packaging, the provision of a large number of doses within a single package is not desirable, as some doses may become unusable due to exposure to the environment. In addition, many drugs are susceptible to a short shelf life when removed from a foil storage pouch or other sealed container, even under nominal environmental conditions. These types of drugs must be used almost immediately after being exposed to the environment.

Various other drugs are also most often taken only in a single or a few doses, or infrequently. These may include vaccines, antidotes, pain reducers, migraine treatments, antivenoms, as well as many others.

Unit dose inhalers are also useful for single dose treatments, non-chronic applications, controlled or very expensive drugs where large quantities of drug would not be acceptable, or for drugs where overdose or abuse would have serious consequences. Unit dose inhalers may also be advantageous for children where providing them with a single dose only avoids the potential for overdosing. Most preferably, a single use inhaler will be disposable, and hence it is particularly preferable that the inhaler is manufactured from recyclable material. It is also preferable that the inhaler is resistant to tampering, both before and after use.

A problem that is common to all forms of inhaler is ingress of moisture into the device. It is important that any inhaler be resistant to the ingress of moisture in order to ensure accurate and uniform dosing. Even low levels of moisture may cause agglomeration of the medicament, resulting in a decrease in the respirable fine particle fraction (FPF).

EP 0629136, EP 0558879 and EP 0548166 describe inhalers comprising a tubular housing having at least two parts that form an air flow path which is open at both ends, one end forming an air inlet and one end forming an air outlet. However, the dose of medicament is stored in a well in the base of the device and entrainment of medicament from that well may not be entirely satisfactory. The devices are also very open in construction and medicament may easily be inadvertently lost, leading to reduced effectiveness. US 6915802 describes a medicament pack that may contain a unit dose for use in a single disposable inhaler. US 5042472 relates to powder medicament compartments which are sealed by a peel-off piece of lidding material. A compartment has two apertures; one over which the mouth of the patient is placed to receive the powder medication by inhalation, and the other of which permits ingress of air to aerosolise the powder medication. US 4841964 relates to inhalers comprising an endless orbital path for one or more balls. The solid substance to be inhaled is provided, preferably in the form of a film, on the surface of the orbital path or on the surface of the ball. A peel-off material seals the inlet and outlet openings.

Inhalation provides several advantages over other delivery techniques such as oral delivery via the mouth or intravenous delivery using a syringe. Inhalation is fast, patient friendly, non-invasive, and can provide rapid absorption into the body. While unit dose inhalers have been proposed in the past, they have met with only varying degrees of success due to performance or other factors as described above. Accordingly, there is a need for an improved unit or single dose inhaler for efficiently providing a prepackaged single dose of a powdered drug.

Accordingly, it is an object of the invention to provide an improved unit or single dose dry powder inhaler and compositions for use therein. We have devised a novel form of inhaler which overcomes or substantially mitigates disadvantages associated with the prior art, and which may be particularly suitable for use as a disposable, single-use device.

According to the invention there is provided an inhaler comprising a first body member and a second body member, the first body member and the second body member fitting closely together to form an inhaler body, and an outlet, the first body member including a medicament chamber containing a unit dose of powdered medicament and the first body member having affixed to it a foil that closes the medicament chamber and which extends outwardly of the inhaler body such that, in use, it can be grasped by a user and withdrawn, fully or partially, from the inhaler body, thereby releasing the powdered medicament from the medicament chamber, and the second body member including a medicament collection well in which the medicament is collected when released, in use, from the medicament chamber, the medicament collection well forming part of an airway communicating with the outlet such that, in use, air can be drawn by inhalation at the outlet along the airway to entrain medicament in the medicament collection well for inhalation by a user.

Preferably, the first body member and the second body member are separate components that are fastened together in order to form the inhaler body.

Conveniently, the first body member and the second body member are hingedly connected.

Advantageously, the first body member and the second body member are formed as a unitary component, being connected by a living hinge or pre-formed fold line.

Preferably, the surfaces of the first and second body members that are brought together to form the inhaler body are substantially planar.

Conveniently, the medicament chamber is formed as a depression in the planar surface of the first body member, and the medicament collection well is formed as a depression in the planar surface of the second body member.

Advantageously, the airway is formed as a depression in the second body member.

Preferably, the outlet has the form of a tubular passageway.

Conveniently, the foil has the form of an elongate strip, one end portion of which is affixed to the surface of the first body member so as to occlude the opening of the medicament chamber.

Advantageously, the foil is folded back on itself, with the free end of the foil extending out of the inhaler body.

preferably, the inhaler is provided with a visual indicator by which, prior to use, a user may verify that the seal between the foil and the first body member is intact.

Conveniently, said visual indicator is an aperture or window through which a part of the sealing foil is visible.

Advantageously, the medicament chamber and the medicament collection well are of comparable dimensions and, after the foil has been removed and the medicament released from the medicament chamber, the medicament chamber and the medicament collection well together form a swirl chamber in which a turbulent airflow is set up during inhalation.

According to another aspect of the invention, there is provided an inhaler comprising an inhaler body including a swirl chamber, the swirl chamber being divided by a foil into a first compartment and a second compartment, the first compartment being sealed by the foil and containing a unit dose of powdered medicament and the second compartment being in communication with an outlet, the arrangement being such that removal of the foil releases the medicament from the first compartment such that, in use, air can be drawn by inhalation at the outlet to entrain medicament in the swirl chamber for inhalation by a user.

Preferably, the medicament chamber or first compartment is substantially hemispherical in form.

Conveniently, the medicament collection well or second compartment is substantially hemispherical in form.

Advantageously, the medicament chamber or first compartment and the medicament collection well or second compartment are substantially hemispherical and have equal diameters, so that together they constitute a substantially spherical swirl chamber.

Preferably, the medicament collection well or second compartment communicates with the outlet via a channel formed in the surface of the second body member.

Conveniently, the channel is of reduced cross-sectional area at its junction with the medicament collection well, so as to form a constriction in the airflow from the medicament collection well to the channel.

Advantageously, the first body member is made of metal.

Preferably, the second body member is made of metal.

Conveniently, the whole of the inhaler body is made from metal.

According to another aspect of the invention, there is provided an inhaler comprising a first body member and a second body member, the first body member and the second body member being formed in metal and fitting closely together to form an inhaler body, and an outlet,
the first body member including a medicament chamber containing a unit dose of powdered medicament and the first body member having affixed to it a foil that closes the medicament chamber and which extends outwardly of the inhaler body such that, in use, it can be grasped by a user and withdrawn, fully or partially, from the inhaler body, thereby releasing the powdered medicament from the medicament chamber,
and the inhaler body including an airway communicating with the outlet such that, in use, air can be drawn by inhalation at the outlet along the airway to entrain medicament released from the medicament chamber for inhalation by a user.

Preferably, the metal is aluminium or steel.

Conveniently, the foil is made of aluminium.

Advantageously, the inhaler contains a single unit dose of a single medicament.

Preferably, the inhaler contains a plurality of unit doses of a single medicament.

Conveniently, the inhaler contains a single unit dose of each of two or more medicaments.

Advantageously, the unit doses of each of the medicaments are contained within the same medicament chamber.

Preferably, the unit doses of each of the medicaments are contained in separate medicament chambers.

According to a further aspect of the invention there is provided an inhaler according to the invention, which contains a plurality of unit doses of each of two or more medicaments.

According to a further aspect of the invention, there is provided a method of delivering a medicament to a patient, which method comprises the inhalation of the medicament from an inhaler according to the invention.

According to another aspect of the invention, there is provided an inhaler comprising a first body member and a second body member, the first body member and the second body member fitting closely together to form an inhaler body, and an outlet,
the first body member including a medicament chamber containing a unit dose of powdered medicament and the first body member having affixed to it a seal member that closes the medicament chamber and which extends outwardly of the inhaler body such that, in use, the seal member can be grasped by a user and withdrawn, fully or partially, from the inhaler body, thereby releasing the powdered medicament from the medicament chamber,
the inhaler body including a dispersion chamber, and the inhaler body including an airway communicating with the outlet such that, in use, air can be drawn by inhalation at the outlet along the airway to entrain medicament released from the medicament chamber for dispersion in the dispersion chamber for inhalation by a user.

Preferably, the second body member includes a medicament collection well in which the medicament is collected when released, in use, from the medicament chamber.

Conveniently, the medicament collection well forms part of the airway, such that, in use, the air drawn by inhalation at the outlet along the airway entrains the medicament in the medicament collection well.

Advantageously, the first body member and the second body member are separate components that are fastened together in order to form the inhaler body.

Preferably, the first body member and the second body member are hingedly connected.

Conveniently, the first body member and the second body member are formed as a unitary component, being connected by a living hinge or pre-formed fold line.

Advantageously, the surfaces of the first and second body members that are brought together to form the inhaler body are substantially planar.

Preferably, the medicament chamber is formed as a depression in the planar surface of the first body member.

Conveniently, the medicament collection well is formed as a depression in the planar surface of the second body member.

Advantageously, the airway is formed as a depression in the first body member and/or second body member.

Preferably, the outlet has the form of a tubular passageway.

Conveniently, the seal member comprises an elongate strip, one end portion of which is affixed to the first body member so as to occlude the opening of the medicament chamber.

Advantageously, the seal member is folded back on itself, with the free end of the seal member extending out of the inhaler body.

Preferably, the inhaler is provided with a visual indicator by which, prior to use, a user may verify that the seal between the seal member and the first body member is intact.

Conveniently, said visual indicator comprises an aperture or window through which a part of the seal member may be viewed.

Advantageously, the medicament chamber and the medicament collection well are of comparable dimensions and, after the seal member has been removed and the medicament released from the medicament chamber, the medicament chamber and the medicament collection well together form a swirl chamber in which a turbulent airflow is set up during inhalation.

Preferably, the medicament chamber is substantially hemispherical in form.

Conveniently, the medicament collection well is substantially hemispherical in form.

Advantageously, the medicament chamber and the medicament collection well are substantially hemispherical and have substantially equal diameters, so that together they constitute a substantially spherical swirl chamber.

Preferably, the medicament collection well communicates with the outlet via a channel formed in the surface of the first body member and/or second body member.

Conveniently, the channel is of reduced cross-sectional area at its junction with the-medicament collection well, so as to form a constriction in the airway from the medicament collection well to the channel.

Advantageously, the first body member is made of metal.

Preferably, the second body member is made of metal.

Conveniently, the whole of the inhaler body is made from metal.

Advantageously, the metal is aluminium or steel.

Preferably, the seal member comprises foil.

Conveniently, the foil comprises aluminium.

Advantageously, the inhaler contains a single unit dose of a single medicament.

Preferably, the inhaler contains a plurality of unit doses of a single medicament.

Conveniently, the inhaler contains a single unit dose of each of two or more medicaments.

Advantageously, the unit doses of each of the medicaments are contained within the same medicament chamber.

Preferably, the unit doses of each of the medicaments are contained in separate medicament chambers.

Conveniently, the inhaler contains a plurality of unit doses of each of two or more medicaments.

Advantageously, the dispersion chamber contains at least one bead, the at least one bead being dimensioned so that it can move around the dispersion chamber.

Preferably, the dispersion chamber comprises a peripheral bead race, and wherein the at least one bead can move about in the bead race.

Conveniently, the dispersion chamber comprises from 2 to 10 beads.

Advantageously, the dispersion chamber comprises a plurality of beads and at least one bead has a shape different from at least one other bead.

Preferably, the dispersion chamber comprises means to cause the at least one bead to move in a chaotic manner.

Conveniently, the means to cause the at least one bead to move in a chaotic manner comprises an obstruction in the dispersion chamber.

Advantageously, the inhaler further comprises means to generate a sheath of air within the periphery of the outlet when air is drawn by inhalation at the outlet.

Preferably, the means for generating the sheath of air comprises a plurality of air inlets in the outlet.

Conveniently, the medicament comprises a triptan.

Advantageously, the triptan is selected from almotriptan, rizatriptan, naratriptan, zolmitriptan, sumatritpan, eletriptan, frovatriptan, and combinations thereof.

Preferably, the triptan is frovatriptan.

According to another aspect of the invention, there is provided a dry powder for inhalation comprising a desiccant and a bioactive agent.

Preferably, the bioactive agent is selected from β₂-agonists, fenoterol, formoterol, pirbuterol, reproterol, rimiterol, salbutamol, salmeterol, terbutaline, non-selective β-stimulants, isoprenaline, xanthine bronchodilators, theophylline, aminophylline, choline theophyllinate, anticholinergics, ipratropium bromide, oxitropium, tiotropium, mast cell stabilisers, sodium cromoglycate, ketotifen, bronchial anti-inflammatory agents, nedocromil sodium, steroids, beclomethasone dipropionate, fluticasone, budesonide, flunisolide, triamcinolone, mometasone, ciclesonide, antivirals, zanamivir, ribavirin, flumist, ruprintrivir, pleconaril, antibiotics, tobramycin, doripenem, pentamidine, promixin, aztreonam, antifungals, amphotericin B, and combinations thereof.

Conveniently, the bioactive agent is selected from immunogens for the prevention or treatment of meningococcal disease, meningitis, septicaemia, meningoccaemia, pneumonia, meningococci of any of groups A, B, C, Y, W135, X and/or Z, anthrax, plague, small pox, tularaemia, meliodosis, Q fever, botulism, typhus, cholera, yellow fever, brucellosis, encephalitis, ricin, salmonella, staphylococcal Enterotoxin B, HIV, HepB, CMV, TB, and combinations thereof.

Advantageously, the bioactive agent is selected from proteinaceous compounds, macromolecules, hormones, mediators, insulin, human growth hormone, leuprolide, alpha-interferon, growth factors, anticoagulants, immunomodulators, cytokines, nucleic acids and combinations thereof.

Conveniently, the bioactive agent is a triptan.

Preferably, the triptan comprises almotriptan, rizatriptan, naratriptan, zolmitriptan, sumatritpan, eletriptan, frovatriptan, and combinations thereof.

Conveniently, the triptan comprises frovatriptan.

Advantageously, the dry powder further comprises a carrier.

Preferably, the dry powder comprises the desiccant and the bioactive agent in a ratio of from 0.1:99.9 to 99.9:0.1 % by weight.

Conveniently, the dry powder comprises the desiccant in an amount of not more than about 50% by weight, preferably not more than about 20 % by weight, more preferably not more than about 10% by weight, and most preferably not more than about 5% based on the weight of the powder.

Advantageously, the desiccant comprises particles exhibiting a similar size distribution and rugosity to conventional DPI lactose.

Preferably, the desiccant comprises particles having a diameter which lies between about 20µm and about 1000µm, more preferably between about 50µm and about 1000µm.

Conveniently, the desiccant comprises particles, substantially all of which (by weight) have a diameter of less than about 300µm, and preferably between about 20µm and about 250µm.

Advantageously, the desiccant comprises particles, at least 90% by weight of which have a diameter of from between about 50µm and about 120µm.

Preferably, the desiccant comprises ammonium chloride, ammonium orthophosphate, ammonium sulfate, barium chloride dihydrate, calcium lactate pentahydrate, copper sulfate pentahydrate, magnesium salicylate tetrahydrate, magnesium sulfate heptahydrate, potassium bisulfate, potassium bromide, potassium chromate, potassium dihydrogen orthophosphate, sodium acetate trihydrate, sodium bromoiridate dodecahydrate, sodium carbonate decahydrate, sodium fluoride, sodium hydrogen orthophosphate dodecahydrate, sodium metaperiodate trihydrate, sodium metaphosphate trihydrate, sodium metaphosphate hexahydrate, sodium sulfite heptahydrate, sodium sulfate heptahydrate, sodium sulfate decahydrate, sodium thiosulfate pentahydrate, zinc sulfate heptahydrate and combinations thereof

Conveniently, the desiccant comprises sodium sulfate and/or calcium lactate.

According to a yet further aspect of the invention, there is provided a method of manufacturing a dry powder of the invention comprising mixing the desiccant with the bioactive agent.

Preferably, the desiccant is spray dried with the bioactive agent.

Conveniently, the method further comprises the step of adding a unit dose of the dry powder to an inhaler.

Advantageously, the inhaler is as defined in accordance with the invention.

According to another aspect of the invention, there is provided an inhaler containing a dry powder as defined in accordance with the invention.

According to a yet further aspect of the invention, there is provided a method of delivering a medicament to a patient, which method comprises the inhalation of the medicament from an inhaler as defined in accordance with the invention.

Preferably, the medicament comprises a dry powder as defined in accordance with the invention.

According to yet another aspect of the invention, there is provided a method of delivering a medicament to a patient, which method comprises the inhalation of a medicament comprising a dry powder as defined in accordance with the invention from an inhaler.

Preferably, the inhaler is as defined in accordance with the invention.

Conveniently, the medicament is for the prevention or treatment of migraines.

Advantageously, the method comprises the step of releasing the medicament from the medicament chamber by withdrawing the foil or seal member, fully or partially, from the inhaler body.

The invention will now be described, by way of example, with reference to the accompanying drawings, in which:
Figure 1 is a perspective view of an inhaler body in an open condition, at a first stage of assembly;
Figure 2 is perspective view of the inhaler body of Figure 1, with the medicament chamber filled, at a second stage of assembly;
Figure 3 shows a third stage of assembly, in which a sealing tape is folded back on itself;
Figure 4 shows the inhaler body immediately after the third stage of assembly;
Figure 5 shows a fourth stage of assembly, in which the inhaler body is folded;
Figure 6 is a perspective view of the assembled inhaler;
Figure 7 is a perspective view of the inhaler, showing the manner in which the inhaler is prepared for use; Figure 8 is a perspective view of an inhaler according to the invention with a peelable seal member conjoined to a mouthpiece cover, which is in a closed position;
Figure 9 is a perspective view of the inhaler of Figure 8 with the mouthpiece cover removed;
Figure 10 is a perspective view of another inhaler of the invention, showing a peelable seal member and separate mouthpiece cover, which is in a closed position;
Figure 11 is a perspective view of the inhaler of Figure 10 with the mouthpiece cover removed;
Figure 12 is a sectional view of the inhaler of Figure 11 along the line A-A;
Figure 13 is a perspective view of the second body member of the inhaler of
Figure 11;

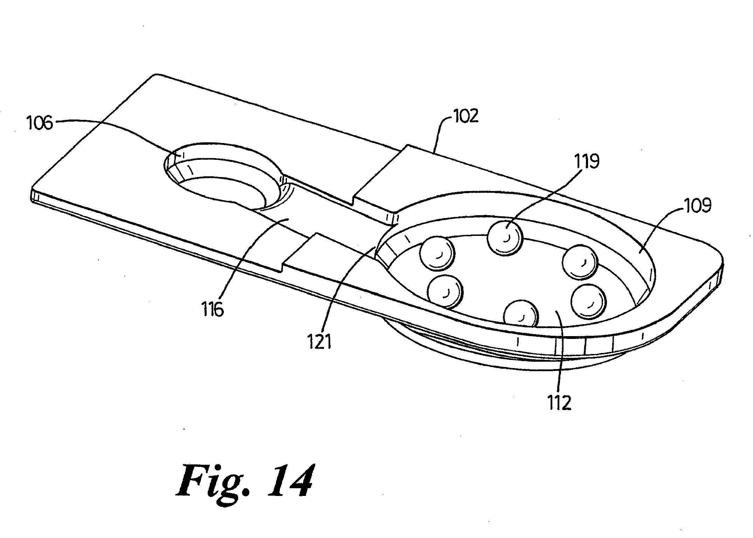

Figure 14 is a perspective view of the second body member of the inhaler of

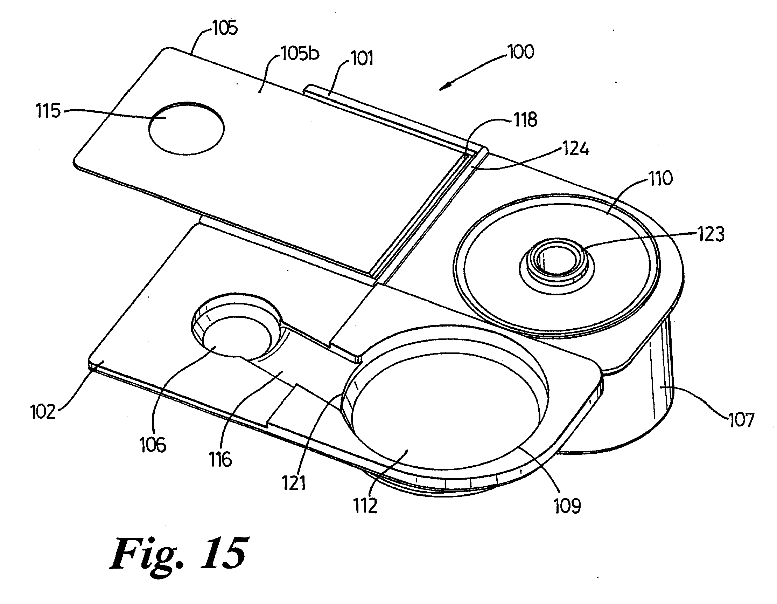

Figure 15 including beads in a dispersion chamber; and

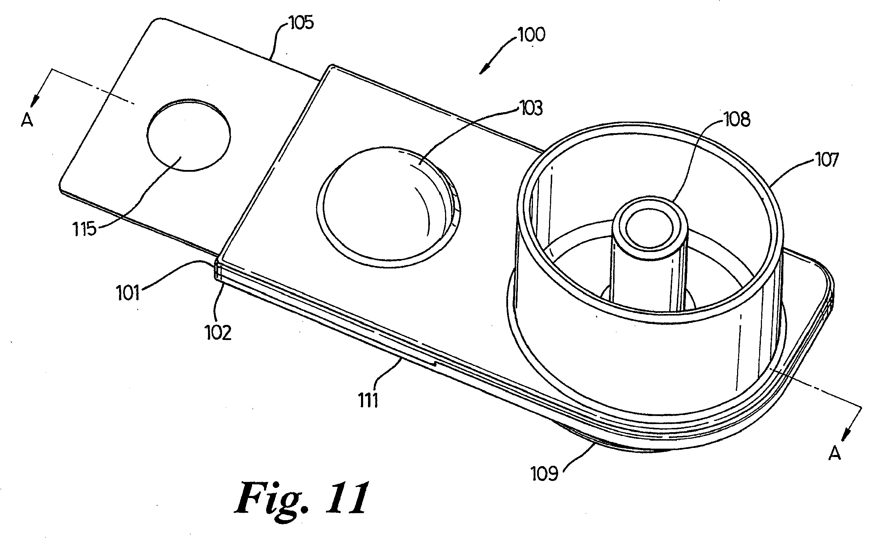

Figure 16 is a perspective of the inhaler of Figure 11 in an open position, prior to inclusion of medicament and without beads, and before sealing for use.

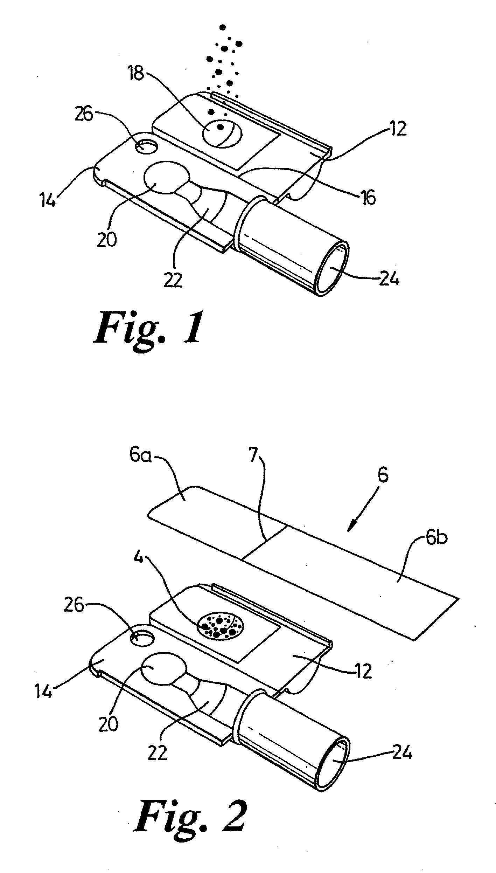

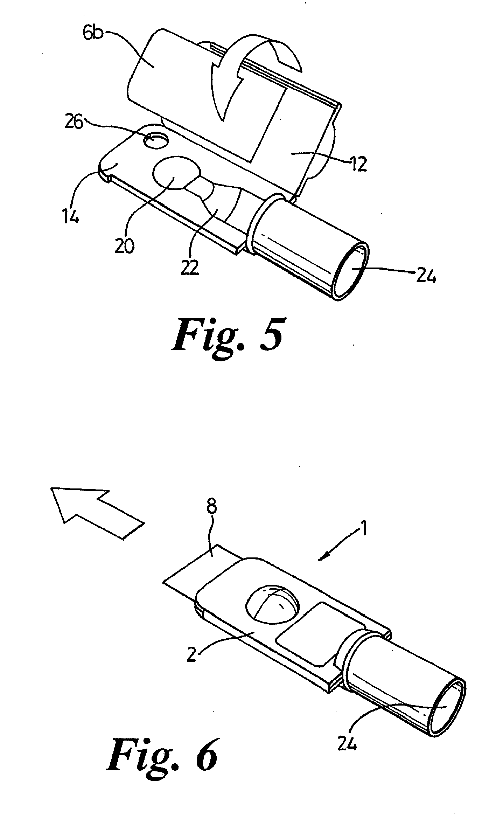

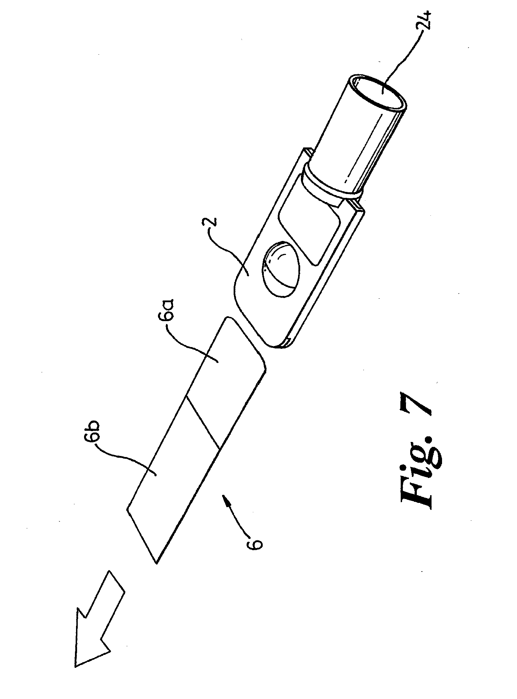

An inhaler according to the invention (shown fully assembled in Figure 6) is generally designated (1) and comprises an inhaler body (2) with an integrally formed outlet (24), a unit dose of a powdered medicament (4) and a sealing foil (6). Figures 1 to 5 show stages in the assembly of the inhaler (1), Figure 6 shows the fully assembled inhaler, and Figure 7 indicates how it is used.

Referring first to Figure 1, the inhaler body (2) comprises first and second cooperating members (12,14 respectively). The first body member (12) and second body member (14) are both generally rectangular in form and are hingedly connected along one of their major edges, the junction between the first body member (12) and the second body member (14) constituting a so-called "living" hinge or pre-formed fold line (16).

The first body member (12) is formed with a hemispherical recess that constitutes a medicament chamber (18).

The second body member (14) also has a generally hemispherical well that in this case constitutes a medicament collection well (20). The medicament collection well (20) is connected by a channel(22) to the tubular outlet (24) that is formed integrally with the second body member (14).

The medicament chamber (18) and the medicament collection well (20) are positioned such that, in the assembled inhaler and as described in greater detail below, medicament may be released from the powder compartment (18) to the medicament collection well (20) and then withdrawn form the medicament collection well (20) via the channel (22) and the outlet (24).

The channel (22) is of reduced dimension at its junction with the medicament collection well (20) such that the junction constitutes a constriction in the path from the medicament collection well (20) to the outlet (24).

At its end that is distal to the outlet (24), the second body member (14) has a circular aperture (26). The aperture (26) is used to indicate the condition of the inhaler (in particular, whether the dose of medicament has been released from the medicament chamber (18)), as described below.

The inhaler body (2) is formed as a unitary component entirely in aluminium or steel, in a pressing operation.

In a first stage of assembly (Figure 1), the medicament chamber (18) is filled with a unit dose of powdered medicament (4). The medicament chamber (18) is then sealed by application to the first body member (12) of the foil (6). The foil (6) is formed of aluminium foil or a plastics-laminated aluminium foil. The foil (6) has a width slightly less than that of the first body member (12), and comprises two portions (6a,6b) that are connected at a crease (7).

The two portions (6a,6b) of the foil (6) are of unequal size such that the portion (6a) is a minor portion and the portion (6b) is somewhat longer.

It is the minor portion (6a) of the foil (6) that is affixed to the surface of the first body member (12). The minor portion (6a) of the foil (6) covers approximately two-thirds of the surface of the first body member (12), the relatively large surface area around the medicament chamber (18) facilitating the formation of a highly effective and moisture-proof seal.

Although attachment of the foil (6) must be secure, to prevent loss of medicament from the medicament chamber (18) and the ingress of moisture, it must nonetheless be possible for a user to remove the foil easily from the inhaler body (2), as described below.

The foil may be attached by means of a relatively weak weld, but is more preferably attached using a suitable adhesive.

The substantially planar nature of the surface of the first body member (12) to which the foil (6) is applied means that there is a large surface area available around the medicament chamber (18) for the foil (6) to be adhered to. Adhesive is preferably applied to the entire surface area around the medicament chamber (18), thus covering a substantial proportion of the surface of the first body member (12).

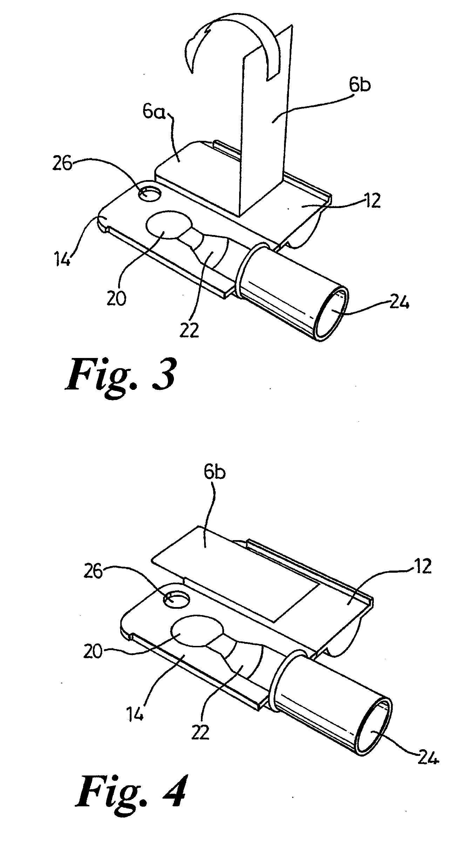

After the minor portion (6a) of the foil (6) has been affixed to the first body member (12), the major portion (6b) of the foil e (6) is folded about the crease (7) (Figure 3) so that it overlies, and projects beyond, the minor portion (6a) (Figure 4).

In a final stage of assembly (Figure 5), the first body member (12) and the second body member (14) are brought together by folding about the hinge (16). The free edges of the first body member (12) and the second body member (14) are formed with cooperating formations that engage with a snap fit, the two components being further fastened together by the application of heat. In this condition, the foil (6) occludes the opening of the medicament chamber (18), thus encapsulating the unit dose (4) of medicament and dividing the medicament chamber (18) from the medicament collection well (20). The foil (6) is received closely in the space between the first body member (12) and the second body member (14), but not so closely that it is completely immobilised.

The fully assembled inhaler (1) is shown in Figure 6. As can be seen, the free end of the folded over major portion (6b) of the foil (6) extends from the inhaler body (2) and forms a tab (8) that can be grasped by a user. A part of the foil (6) is visible through the aperture (26) and carries a visual indicator, eg a particular colour, number or other symbol, that confirms to a user that the foil (6) is in its operative position and hence that the dose of medicament has not previously been exposed.

Although the foil (6) effectively protects the unit dose of medicament (4) from moisture, for additional protection the assembled inhaler (1) may be supplied in secondary packaging, eg a sealed sachet such as a conventional laminated foil sachet.

To use the inhaler (1), a user first removes it from any secondary packaging. The tab (8) is then grasped and pulled away from the inhaler body (2). The foil (6) is thereby peeled away from the first body member (12) and released from the inhaler body (2) (see Figure 7).

The unit dose of medicament (4) is thus released from the medicament chamber (18) and falls into the medicament collection well (20). With the foil (6) removed, the medicament chamber (18) and the medicament collection well (20) together constitute a substantially spherical swirl chamber. The user places the outlet (24) between their lips and inhales through it. Air is drawn into the inhaler body (2) via the gap through which the sealing tape (6) was withdrawn. Medicament in the medicament collection well (20) is entrained in the airflow, undergoes turbulent flow within the swirl chamber made up of the medicament chamber (18) and the medicament collection well (20), is drawn out of the inhaler body (2) via the channel (22) and the outlet (24), and is inhaled by the user.

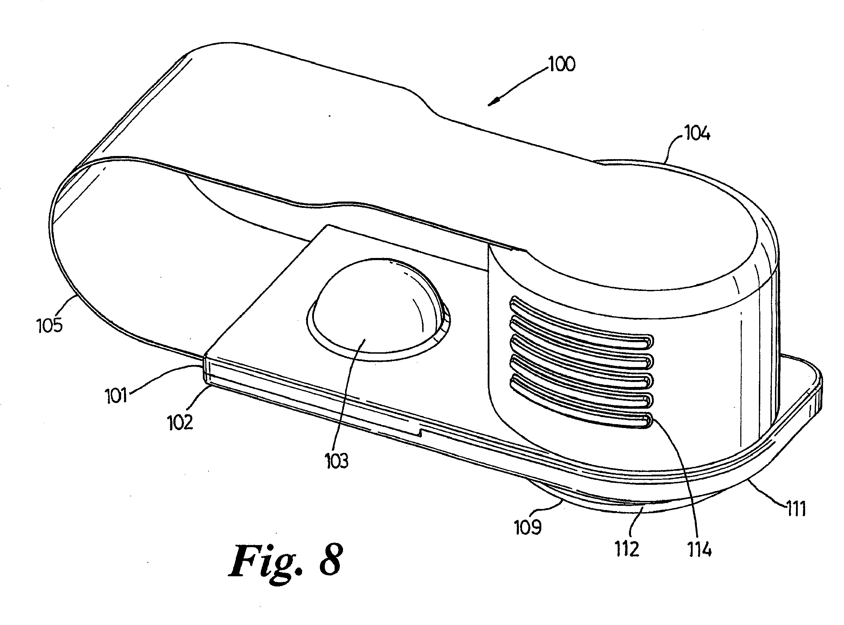

Turning to Figure 8, a fully assembled inhaler according to the invention is shown generally designated (100). The inhaler (100) comprises a first body member (101) and second body member (102), which are both generally rectangular in form. The first body member (101) and second body member (102) cooperate to form an inhaler housing (111).

The first body member (101) is formed with a hemispherical recess that constitutes a medicament chamber (103) for containing a unit dose of medicament. An outlet in the form of a mouthpiece (107) is also formed in the first body member (101), which is not visible in Figure 8. As will be described below, the mouthpiece comprises two concentric annular walls which project outwards from the outer face of the first body member (101). Figure 8 shows the mouthpiece (107) covered by a mouthpiece cover (104) in order to minimise risk of contamination of the inhaler (100) before use. The mouthpiece cover (104) is in the close position, obscuring mouthpiece (107) and air/powder outlet (mouthpiece tube) (108). The mouthpiece cover (104) has grip areas (114) formed on the sides.

The second body member (102) has a generally disk-like recess (not seen in this view) that forms a medicament collection well (106). The medicament collection well is located in registration with the medicament chamber when the first and second body members (1, 2) are assembled to form the inhaler housing (111)). A dispersion chamber (109) is partly formed in the second body member (102) by a base chamber wall (112). A seal member (105) of slightly reduced width than the first body member (101) extends from within the inhaler housing (111) and is connected to the upper surface (113) of the mouthpiece cover (104). As will be explained below, the seal member (105) is attached to the first body member (101) to seal the medicament within the medicament chamber (103). In use, the seal member (105) may be withdrawn from the inhaler housing (111) to release the medicament from the medicament chamber (103), so that it may fall into the medicament collection well (106). An airway is formed within the inhaler housing (111) from a gap between the first and second body members (101, 102) where the seal member (105) projects, through the medicament collection well (106), into the dispersion chamber and out through the outlet (108) within the mouthpiece (107).

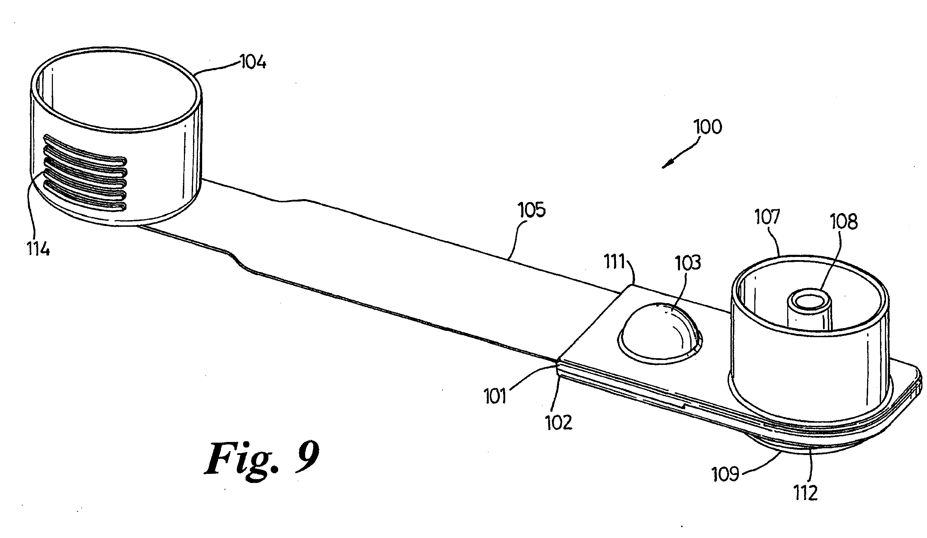

Figure 9 shows the inhaler (100) with the mouthpiece cover (104) removed from the mouthpiece (107). The mouthpiece (107) surrounds the annular air/powder outlet (mouthpiece tube) (108), which leads to the dispersion chamber (109). A user may pull on the mouthpiece cover (104) to withdraw the seal member (105) fully or partially from the inhaler housing (111) to release the medicament from the medicament chamber (103) into the medicament collection well (106) ready for inhalation.

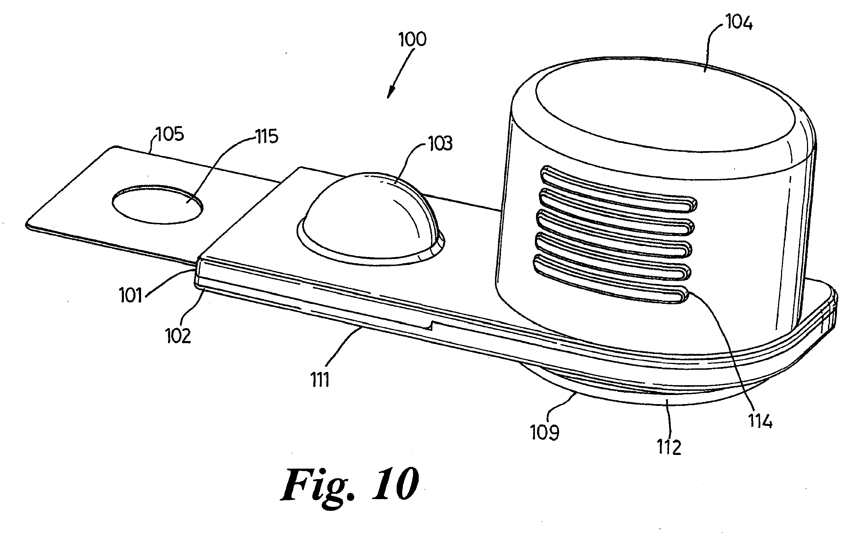

Figure 10 shows an inhaler similar to that shown in Figure 8 except that the seal member (105) is not connected to the mouthpiece cover (104). Also, a circular aperture (115) in the seal member forms an aid to the grip and peel action of the user in withdrawing the seal member (105) from the inhaler housing (111) to release the medicament ready for inhalation.

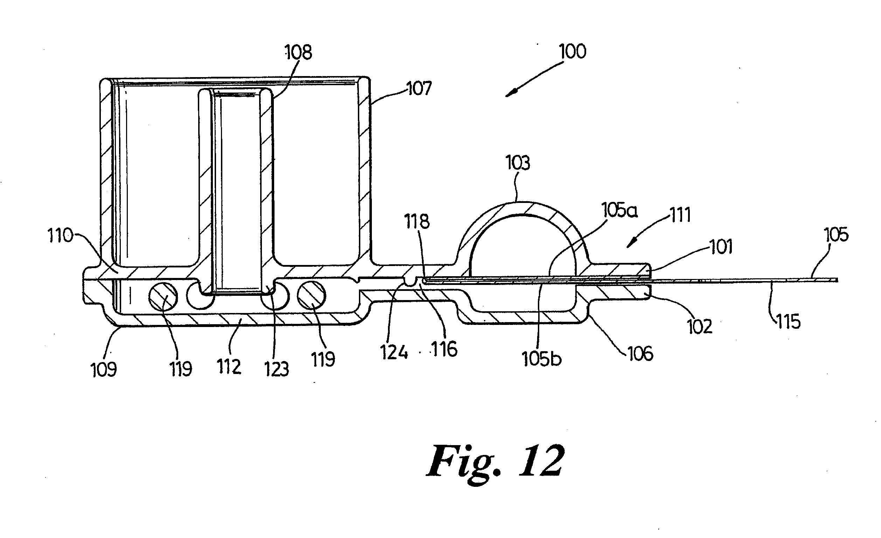

Figure 12 is a sectional view of the inhaler of Figure 11 along the line A-A and shows the internal detail of this embodiment. The second body member (102) is formed with a circular disk-shaped recess that constitutes the medicament collection well (106). The medicament collection well (106) and the dispersion chamber (109) are connected via an airway (channel) (116) formed in the second body member (102). The airway (116) leads from the end of the inhaler housing (111) adjacent to the protruding portion of the seal member (105), into the medicament collection well (106). The airway then leads to the dispersion chamber (109), passing a ridge (124) projecting from the lower surface of the first body member into the airway (116). The ridge (124) forms a constriction in the airway (116). The airway leads from the constriction (124) into the dispersion chamber (109), and out of the inhaler housing (111) though the air/powder outlet (108).

The medicament chamber (103) and the medicament collection well (106) are positioned such that, in the assembled inhaler and as described in greater detail below, medicament may be released from the powder compartment (117) to the medicament collection well (106) and then withdrawn from the medicament collection well (106) via the channel (116) and the dispersion chamber (109). The seal member 5 comprises two portions (105a, 105b) which are of unequal size. The portion (105a) is attached to the first body member (110) and is of shorter length than the remaining portion (105b) which extends out of the housing (111). The minor portion (105a) of the seal (105) is attached to the major portion (105b) at a crease (118) located between the medicament chamber (103) and the mouthpiece (107). In the embodiment shown in Figure 12, the minor portion (105a) of the seal member (105) covers approximately two-thirds of the surface of the portion of the first body member (101) surrounding the medicament chamber (103). This gives a relatively large sealing area around the medicament chamber (103) facilitating the formation of a highly effective and moisture-proof seal.

Although attachment of the seal member (105) must be secure, to prevent loss of medicament from the medicament chamber (103) and the ingress of moisture, it must nonetheless be possible for a user to remove the seal member easily from the inhaler body (111), as described below.

The seal member may be attached by means of a relatively weak weld, but is more preferably attached using a suitable adhesive. The substantially planar nature of the surface of the first body member (101) to which the seal member (105) is applied means that there is a large surface area available around the medicament chamber (103) for the seal member (105) to be adhered to. Adhesive is preferably applied to the entire surface area around the medicament chamber (103), thus covering a substantial proportion of the available planar surface of the first body member (101). After the minor portion (105a) of the seal member (105) has been affixed to the first body member (101), the major portion (105b) of the seal member (105) is folded about the crease (118) so that it overlies, and projects beyond, the minor portion (105a). Thus, when the user pulls on the end of the major portion (105b) of the seal (105), the minor portion (105a) is gradually peeled away from the first body member (101) in a line from beyond the medicament chamber (103) towards the end of the housing (111). In doing so, the seal member (105) is removed from the medicament chamber (103), releasing the medicament so that it may fall into the medicament collection well (106).

The dispersion chamber (109) is formed within the inhaler housing (111) by the shape of the first body member (101) and the second body member (102). In particular, the first body member defines a generally annular top chamber wall (110), and the second body member defines a generally annular base chamber wall (112).

One or more beads (119) are contained within the dispersion chamber (109) as described in US Patent Numbers US 6,427,688; US 6,715,486; and US 6,971,384; and International Patent Application PCT/US01/03248, all of which are incorporated herein by reference.

An annular ridge or ring (123) projects from the top chamber wall (110) of the second body member (102) towards the base chamber wall (112) of the first body member, leaving a gap between the distal end of the ring (123) and the base chamber wall (112).

The beads (119) are dimensioned such that they may move around the dispersion chamber (109), but may not pass into the airway (116) or through the gap under the retaining chamber ring (123) and into the air/powder outlet (108).
When air is drawn through the inhaler by the user, the air flows through the medicament collection well (106), entraining the medicament powder and carrying it along the airway channel (116). The flow of medicament powder and air enters the dispersion chamber (109), preferably at an angle to the plane and/or axis of the dispersion chamber (109). The turbulent flow of air within the dispersion chamber (109) helps to disperse the medicament in the flow of air to form an effective aerosol of medicament. The dispersed medicament is entrained out of the dispersion chamber through the powder/air outlet and into the user's body. When beads (119) are present in the dispersion chamber (109), the flow of air causes them to move about within the dispersion chamber (109), helping to form the aerosol of medicament. However, the beads (109) are dimensioned such that they cannot pass into the airway channel (116) or through the gap defined by the chamber ring (123).

Sheath air inlets (not shown), if present, may preferably extend radially inwardly to a ring in the air/powder outlet (mouthpiece tube) (108) to provide an annular flow of sheath air surrounding the flow of powder-laden air in the mouthpiece (107). Sheath air inlets are described in US Patent Numbers US 6,427,688; US 6,715,486; and US 6,971,384; and International Patent Application PCT/US01/03248, all of which are incorporated herein by reference. Sheath air inlets help to minimise the deposition of particles on the walls of the air/powder outlet and so promote efficient inhalation of the medicament. The air/powder outlet (mouthpiece tube) (108) extends from a central area of the top chamber wall (110) to or through the mouthpiece (107).

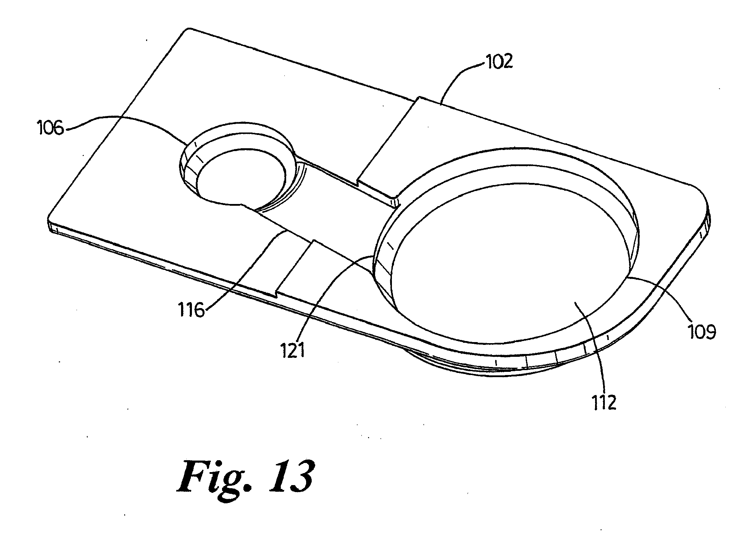

Figure 13 shows the second body member (102). The second body member (102) is formed with a circular disk-shaped recess that constitutes the medicament collection well (106). The airway (channel) (116) is of reduced dimension at its junction with the medicament collection well (106) such that the junction constitutes a constriction in the path from the medicament collection well (106) to the dispersion chamber (109). The inlet (121) is non-angled relative to the bead chamber (120) and is in the form of a chord inlet, entering the bead chamber (120) non-tangentially along a chord. Alternatives to inlet features are described in US Patent Numbers US 6,715,486 and US 6,971,384; and International Patent Application PCT/US01/03248, all of which are incorporated herein by reference. The second body member (102) is also formed with a recess that constitutes the base chamber wall (112) of the dispersion chamber (109). Figure 14 shows the second body member (102) with six beads (119) in the dispersion chamber (109).

Figure 15 shows a stage in the assembly of the inhaler (100). The first body member (101) and second body member (102) are both generally rectangular in form and are hingedly connected along one of their major edges, the junction between the first body member (101) and the second body member (102) constituting a so-called "living" hinge or pre-formed fold line (122). On the second body member (102) the inner surfaces of the medicament collection well (106) and base chamber wall (112) are visible. On the first body member (101) the inner surface of the top chamber wall (110) is visible. Also shown is the minor portion (105a) of the seal member (105), affixed to the first body member (101), the major portion (105b) of the seal member (105), and the crease (118). The major portion (105b) of the seal member (105) overlies the minor portion (105a) and projects beyond the inhaler housing minor portion (111).

In the assembly of the inhaler (100), the medicament chamber (103) is filled with a unit dose of powdered medicament (117). The medicament chamber (103) is then sealed by application to the first body member (101) of the seal member (105). The seal member (105) is formed of aluminium foil or a plastics-laminated aluminium foil. The seal member (105) has a width slightly less than that of the first body member (101), and comprises two portions (5a, 5b) that are connected at a crease (118). In a further stage of assembly, the first body member (101) and the second body member (102) are brought together by folding about the hinge (122). The free edges of the first body member (101) and the second body member (102) are formed with cooperating formations that engage with a snap fit, the two components being further fastened together by the application of heat. In this condition, the seal member (105) occludes the opening of the medicament chamber (103), thus encapsulating the unit dose (117) of medicament and dividing the medicament chamber (103) from the medicament collection well (106). The seal member (105) is received closely in the space between the first body member (101) and the second body member (102), but not so closely that it is completely immobilised. Although the seal member (105) effectively protects the unit dose of medicament (117) from moisture, for additional protection the assembled inhaler (100) may be supplied in secondary packaging, eg a sealed sachet such as a conventional laminated foil sachet.

Thus, according to a first aspect of the invention, there is provided a unit dose inhaler comprising a first body member and a second body member, the first body member and the second body member fitting closely together to form an inhaler body, and an outlet, the first body member including a medicament chamber containing a unit dose of powdered medicament and the first body member having affixed to it a sealing mechanism or foil that closes the medicament chamber and which extends outwardly of the inhaler body such that, in use, it can be grasped by a user and withdrawn, fully or partially, from the inhaler body, thereby releasing the powdered medicament from the medicament chamber, and the second body member including a medicament collection well in which the medicament is collected when released, in use, from the medicament chamber, the medicament collection well forming part of an airway or air flow path communicating with the outlet such that, in use, air can be drawn by inhalation at the outlet.

The medicament chamber may hold a powder formulation containing smaller active pharmaceutical particles, and optionally also containing larger inert carrier particles. According to another aspect of the invention the inhaler includes a dispersion or bead chamber which includes a bead race, and a nosepiece or mouthpiece which has at least one outlet opening connecting or entering into the dispersion chamber. One or more inlets connect into the dispersion chamber from the medicament chamber and/or medicament collection well, adjacent to or in close proximity to the dispersion chamber. The dispersion chamber contains one or more beads which can move about in a bead race. While preferred, the beads are not an essential element.

An airway (air flow path or channel) extends past and/or through and/or under the medicament chamber and into the dispersion chamber. A pharmaceutical dry powder formulation is released from the medicament chamber and entrained in air flow through the inhaler when the user inhales on the mouthpiece. The powder is dispersed in air within the dispersion chamber and forms an aerosol inhaled by the user.

The inhaler according to the invention is advantageous primarily in that it is of simple construction and is therefore relatively easy and inexpensive to manufacture, and is easy to use. In preferred embodiments, the inhaler is particularly small and compact, and is manufactured from recyclable materials. In preferred embodiments, all components of the inhaler are manufactured from the same materials, for example, the same polymer type. In yet further preferred embodiments, all or some components of the inhaler are manufactured from easily recyclable material, for example cardboard or metal. Also, preferred embodiments of the invention provide particularly effective protection of the powdered medicament from the effects of moisture. The performance of the inhaler, eg in terms of the fine particle fraction (FPF) of the medicament delivered to the user, may therefore be good. Also, because the medicament that is released from the medicament chamber is collected in a medicament collection well that forms part of the airway, inadvertent loss of medicament prior to, or during, inhalation is minimised.

The first body member and the second body member may be separate components that are fastened together in order to form the inhaler body. The first body member and the second body member may, for instance, have a snap or interference fit. More preferably, however, the first body member and the second body member are connected, and the first body member and the second body member are brought together by folding. It is particularly preferred that the first body member and the second body member are formed as a unitary component, being connected by a so-called living hinge or pre-formed fold line. Generally, the hinge or fold line will be constituted by material of reduced thickness. The inhaler body can then be assembled by folding about the hinge.

The first and second body members preferably have formations that cooperate to provide a snap or interference fit. The first and second body members may be further secured together, eg by adhesive, or more preferably by the application of heat.

For ease of manufacture, the surfaces of the first and second body members that are brought together to form the inhaler body are preferably substantially planar. The medicament chamber is formed in the first body member, most preferably as a depression in the planar surface of the first body member, and the medicament collection well is formed in the second body member, most preferably as a depression in the planar surface of the second body member. The airway is most preferably formed as a depression, or cooperating depressions, in one or both of the first and second body members, most preferably in the second body member. The dispersion chamber is most preferably formed as a depression, or cooperating depressions, in one or both of the first and second body members. In certain embodiments, the dispersion chamber may be formed exclusively in either the first body member or the second body member.

The abutting faces of the first and second body members are most preferably of substantially equal dimensions, and in particular are most preferably generally rectangular.

The outlet preferably has the form of a tubular passageway. The outlet may be tapered such that its internal dimension increases along the direction of airflow. Such an arrangement may lead to deceleration of the airflow and hence reduce undesirable deposition of the medicament in the user's mouth and upper airway. The outlet may be connected to, or formed integrally with, the first body member or the second body member. Alternatively, the outlet may be formed from components that are connected to, or formed integrally with, the first body member and the second body member and which are brought together to form the outlet when the first body member and the second body member are brought together to form the inhaler body.

The outlet is most preferably dimensioned and configured to facilitate its presentation to a user's mouth, and therefore may generally be considered to be a mouthpiece. However, although inhalers of the present invention will most commonly be used to deliver medicament by inhalation via the buccal cavity (mouth), it will be appreciated by those skilled in the art that inhalation via the nose is also possible. References herein to "mouth" and "mouthpiece" and the like should be construed accordingly, ie to include components adapted to channel inspired air to the patient's airways via the nasal passages, rather than via the mouth.

When a patient inhales on the mouthpiece, air and powder are drawn into, or flow about within, the dispersion chamber. The beads, when present, collide with the interior chamber surfaces, and/or each other, and the powder particles on the chamber surfaces or on the beads. The movement of the beads separate the smaller active drug particles from each other and/or the larger inert carrier particles, if any. In addition to these mechanical forces, other causes of dispersion may include fluid shear between the beads, the powder particles, and the chamber walls. Larger carrier particles, if included in the powder formulation, can further enhance dispersion via enhanced impact energy and abrasion. The active particles are entrained into the airflow through the dispersion chamber, for inhalation by the patient. The larger inert or excipient carrier particles may or may not be entrained and inhaled. The carrier particles are advantageously provided to scour the powder path clean of the fine active particles, so that a more uniform dose may be delivered.

In a further embodiment of the invention, the beads within the dispersion chamber are induced to move chaotically, so that most or all of the interior surfaces of the dispersion chamber, and the surfaces of the beads are contacted. As a result, less of the powder may be held up within the dispersion chamber, and a more uniform dose may be delivered. Flow rate performance may also be improved.

In a yet further embodiment of the invention, the flow resistance of a dry powder inhaler is reduced by providing one or more beads into the air flow path of the inhaler. As a result, improved dispersion of powder is achieved, with no additional inspiratory effort by the patient.

A bead is a loose component not physically attached to any other component or surface of the inhaler, so that it is free to move within the inhaler, with at least one degree of freedom. A bead race is a surface, which a bead contacts, continuously or intermittently. A bead race may be a well-defined or consistent path in or on which beads uniformly move about, or it may be a surface not part of such a path. The bead race may reside within the limits of the first body member. Alternatively the bead race may reside within the limits of the second body member. Preferably, the bead race resides within the limits of both the first and second body members.

A dispersion chamber is a chamber or confined area wherein dry powder is dispersed and/or mixed with air. The dispersion chamber may be the only location where powder is dispersed, or it may be one of two or more such locations for powder dispersing or deagglomerizing features. In certain embodiments of the invention, powder dispersion or deaggregation may occur, or may occur in addition, prior to the dispersion chamber in the air flow path between the medicament chamber and the dispersion chamber and/or in the medicament chamber and medicament well once the powder is released. In certain embodiments of the invention, powder dispersion or deaggregation may occur, or may occur in addition, after the dispersion chamber in the air flow path between the dispersion chamber and the mouthpiece.

Sheath air is ambient air flow used to reduce particle deposition in the mouthpiece. Sheath air is air drawn into the mouthpiece without passing through the powder flow path of the inhaler. While use of sheath air reduces particle hold up in the mouthpiece, it also reduces the amount of airflow available to move powder through the inhaler for inhalation. Consequently, the flow split between the sheath air path and the powder air path should be appropriately balanced. Typically, the flow split will range from about 30-95%, or more preferably 40-70% of the total air flow (as inspired by the patient) moving through the powder flow path, with the balance moving through the sheath air path. Both flows combine within the inhaler mouthpiece to for the total flow through the inhaler, as generated by the patient's inspiration.

Preferably, sheath air inlets preferably extend radially inwardly to a ring in the mouthpiece tube, to provide an annular flow of sheath air surrounding the flow of powder laden air in the mouthpiece. The outer mouthpiece wall may be longer than the wall of the inner air/powder outlet. This can provide an outer approximately annular region of axial sheath air flow to limit physical contact and deposition of dispersed particles to the walls of the mouthpiece. The sheath airflow increases the efficiency of powder transfer from the inlet to the outlet of the mouthpiece.

Dispersed particles released into a mouthpiece have trajectories not directed solely toward the outlet of the mouthpiece. Further, these particles are often emitted from an area smaller than the cross-section of the mouthpiece. This leads to air flow turbulence and back eddies that can lead to particle deposition inside the mouthpiece. Consequently, it is important for the mouthpiece to efficiently transfer particles from the point of dispersion to the mouthpiece outlet (which is placed in the patient's mouth).

A sheath air mouthpiece may provide a continuous or near-continuous approximately annular sheath of airflow directed axially through and out of the mouthpiece. The sheath air is not drawn from the dispersion chamber or other region where particles are generated or dispersed. This is intended to provide sheath air, which is largely free of the pharmaceutical particles being dispersed.

The velocity of the sheath air is preferably approximately matched to, and not excessively greater than, the velocity of the air flowing into the mouthpiece from the dispersion chamber outlet.

The mouthpiece may have an expanding cone shape, in which the sheath air flows forward (towards the patient's mouth), and also expands along the cone. The cone angle is made gradual to reduce the effects of flow separation and resulting pressure drop and particle deposition.

If, due to flow characteristics, the dry powder pharmaceutical particles flow out of the dispersion chamber towards the walls of the mouthpiece, the design may be modified to provide a thicker layer of sheath airflow between the particles and the wall. This may be accomplished by asymmetrically varying the thickness of the thickness of the sheath air annulus to create a thicker sheath air flow in regions where particles would otherwise contact and settle out in the mouthpiece. The thicker layer of sheath air is provided to absorb and redirect the particles flowing in trajectories towards the interior mouthpiece walls, and to limit contact between the particles and the walls of the mouthpiece. The sheath air annulus need not be ring shaped, like a true geometric annulus. It may be flattened, with thicker side lobes connected by thinner web sections. This can be done preferably by having an exit tube with a round outside surface surrounded by inner walls of the mouthpiece tube shaped in an ellipse, oval, or other flattened or elongated curved shape.

The air/powder outlet into the mouthpiece need not be centred in the mouthpiece inlet. It may be off-centre with the optionally thickened sheath air layer introduced between the air/powder outlet and the cylindrical or conical wall of the mouthpiece.

Tests on mouthpieces with sheath air show improved performance relative to the conventional inhaler design. Hold-up within the mouthpiece was reduced to about 30 to 50% of the hold-up in an equivalent inhaler without sheath air. Computational fluid dynamics models indicate that particle deposition within the mouthpiece may be reduced by 70% or more using sheath air. Hold-up is the amount of medicament remaining in the mouthpiece after use.

The advantages of providing sheath air include: increased particle delivery efficiency, reduced priming effect, reduced cleaning requirements, increased dosing precision, reduced manufacturing costs (e.g., less drug required), and greater aerosolization efficiency in the sheath air shear field. The priming effect is the tendency for initial doses to be reduced due to deposition on the mouthpiece surfaces.

While the description of sheath air above is made primarily with reference to the specific inhalers shown in the drawings, the features and principles described apply as well to any inhaler having a mouthpiece, regardless of the dispersion mechanism, powder or other drug media storage and release technique, and powder or drug media flow or movement designs used.

In yet another aspect of the invention, the mouthpiece has an optional mouthpiece cover or cap which is removable from the mouthpiece. Optionally the mouthpiece cover or cap is attached to the sealing mechanism of the medicament chamber.

The medicament container remains sealed until the inhaler is ready for use. The medicament chamber is filled with a unit dose of powdered medicament, and then the medicament chamber is sealed by the seal member. It is preferable that the medicament chamber be substantially completely filled, in order to minimise the risk of exposure to moisture that might be present in any trapped air or other gas. The seal member most preferably has the form of an elongate strip, one end portion of which is affixed to the surface of the first body member so as to occlude the opening of the medicament chamber. The seal member may be affixed to the surface of the first body member by any suitable means, eg by means of a relatively weak weld, but is most preferably affixed by means of a suitable adhesive. Although attachment must be secure to prevent loss of medicament from the medicament chamber and the ingress of moisture, it must nonetheless be possible for a user to remove the seal member so as to release the dose of medicament. A further advantage of a planar surface of the first body member around the medicament chamber is that it facilitates secure attachment of the seal member to that surface, particularly if there is a substantial planar area around the opening of the medicament chamber.

The seal member extends from the inhaler body. The length of the seal member is such that the free end, which extends out of the inhaler body, forms a suitably sized tab that can be gripped between the user's fingers for partial or full removal of the seal member. Preferably, the seal member is configured so that the tab is at the substantially opposite end of the inhaler to the outlet. It is particularly preferred that the seal member be folded back on itself, with the free end of the seal member extending out of the inhaler body. In this arrangement, when the seal member is grasped and pulled by the user, it is peeled away from the opening of the medicament chamber in a controlled manner, reducing the force required to remove the seal member and minimising the risk of the seal member rupturing during removal. The end of the seal member may be knurled to improve grip and peel in use. Optionally the end of the seal member may include an aperture or apertures to improve the grip and peel action in use.

When the seal member is peeled away from the medicament chamber it may either be removed entirely or it may remain attached to the inhaler body. For example, the end part of the seal member may be fixed more strongly to the surface of the first body member, so that the seal member remains attached after the medicament chamber is opened.

Alternatively the medicament chamber is opened following, or as part of the sequence of, removal of the mouthpiece cover. In another preferred embodiment the seal member forms part of and/or is connected to the mouthpiece cover. The mouthpiece cover is removed and the seal member is peeled from the medicament chamber aided by the additional grip afforded by the conjoined cover. This embodiment will reduce the number of separate components, aid effective and complete disposal, and provide a guide to the sequence of operation.

Step-by-step numbering of the actions required to use the inhaler may be included in the form of embossing or labelling or other methods well understood in the art. It will be understood that the medicament chamber remains sealed until the inhaler is ready for use. As a result, the dose of powder remains sealed within the medicament chamber until moments just before inhalation. The disadvantages of prolonged exposure of the powder to the environment, such as oxidation, particle size growth, caking, etc. are reduced or avoided.

The inhaler with the dose unit is very easy to use as it requires only a single and simple movement to open the medicament container. Preferably, the inhaler is also provided with a visual indicator by which, prior to use, a user may verify that the seal between the seal member and the first body member is still intact. Preferably, the inhaler body, most preferably the second body member, is provided with an aperture or window through which a part of the seal member is visible. That part of the seal member is preferably provided with a visual indicator, eg a particular colour, number or other symbol, that is displaced from the window when the seal member is removed or partially removed. Before use, the user would be able to verify that the seal is still intact and therefore that the medicament is safely sealed in the medicament chamber and is suitable for inhalation. Once the seal member has been pulled, the user would then be able to visually verify that the seal member had been sufficiently withdrawn to release the medicament from the medicament chamber.

The medicament chamber is most preferably formed as a depression or recess in the surface of the first body member. The medicament collection well is likewise preferably formed as a depression or recess in the surface of the second body member. In order for medicament released from the medicament chamber to be collected in the medicament collection well, the medicament chamber and the medicament collection well need to be aligned. In one embodiment, the medicament chamber and the medicament collection well are of comparable dimensions. After the seal member has been removed and the medicament released from the medicament chamber, the medicament chamber and the medicament collection well together may form a swirl chamber in which a turbulent airflow is set up during inhalation. It is possible thereby to promote deagglomeration of the medicament particles and improve the FPF in conjunction with the action of the dispersion chamber. In this embodiment, the swirl chamber is divided by the seal member into two compartments, one being sealed and containing the unit dose of medicament and the other communicating with the outlet. This feature represents a further aspect of the present invention, according to which there is provided an inhaler comprising an inhaler body including a swirl chamber, the swirl chamber being divided by a seal member into a first compartment and a second compartment, the first compartment being sealed by the seal member and containing a unit dose of powdered medicament and the second compartment being in communication with an outlet, the arrangement being such that removal of the seal member releases the medicament from the first compartment such that, in use, air can be drawn by inhalation at the outlet to entrain medicament in the swirl chamber for inhalation by a user.

In preferred embodiments of the invention, the medicament chamber (first compartment) is substantially hemispherical in form. Similarly, the medicament collection well (second compartment) is preferably substantially hemispherical in form. It is particularly preferred that both the medicament chamber and the medicament collection well are substantially hemispherical and that they have equal diameters, so that together they constitute a substantially spherical chamber. In other embodiments, the chamber may be elongated to form a tube, preferably of circular cross-section and with hemispherical ends, or flattened to form a disc-like shape, again preferably of circular cross-section and with a semicircular cross-section at its periphery. In yet further embodiments, the medicament chamber (first compartment) and medicament collection well (second compartment) are of different sizes, the medicament chamber normally being the smaller of the two. In a yet further embodiment, the medicament chamber (first compartment) and medicament collection well (second compartment) are of different sizes, the medicament chamber normally being the larger of the two. In a still further preferred embodiment, the medicament chamber (first compartment) is substantially hemispherical and medicament collection well (second compartment) is formed of a disc-like shape. It will be understood that in this latter embodiment the compartments may be of different volumes but share the same diameter.

The medicament chamber or medicament collection well or both may also be configured in such a manner as to divide the airflow passing through it, eg as described in WO 2004/103446.

It will also be understood that in certain embodiments it will be preferable for the siting of the medicament chamber and medicament collection well to be reversed; the powdered medicament being disposed initially in the depression in the second body member. In this embodiment the seal member most preferably has an end portion of which is affixed to the surface of the second body member so as to occlude the opening of the medicament chamber.

Air may be drawn into the airway via the space through which the seal member is removed from the inhaler housing. Typically such a space has the form of a slot with a width slightly in excess of that of the seal member and a height that is sufficient to permit withdrawal of the seal member but is not so great as to permit any significant inadvertent loss of powder from within the inhaler body. Most preferably, the slot has a height of less than 2mm, more preferably less than 1mm.

Additional air inlets to the medicament collection well may be provided, eg to help generate turbulent airflow in the swirl chamber or to help with airflow generally. Such inlets are again preferably sufficiently small as not to permit any significant loss of powder. Such additional air inlets may lead from edges of the inhaler housing other than that through which the seal member projects.

The medicament collection well preferably communicates with the dispersion chamber and the outlet via a channel or channels formed in the surface of the second body member. Preferably, the channel is of reduced cross-sectional area at its junction with the medicament collection well, so as to form a constriction in the airflow from the medicament collection well to the channel. The channel may have a tortuous form to induce further deagglomeration of the entrained medicament particles. The channel which forms the inlet to the dispersion chamber may be straight, tangential, or non-angled relative to the bead race or bead chamber. Alternative inlet designs may be used to enhance performance.

In one embodiment, the airway may be angled relative to the plane of the dispersion chamber. In other words, the airway may enter the dispersion chamber at an angle relative to the plane of the first and second body members.

Also, the airway may enter the dispersion chamber at an angle and/or position relative to the central axis of the chamber. In one embodiment, the airway may enter the dispersion chamber at a scoop inlet, which has an inner edge or wall that is tangent to the outer diameter or surface of the bead race. Alternatively, the airway may enter at a chord inlet into the dispersion chamber non-tangentially along a chord. The inlets may be non-angled, i.e., extending in the plane of the bead chamber, or they may be angled relative to the plane of the dispersion chamber.

In the scoop inlet embodiment, the outside wall of the inlet is positioned to the outside of a tangent to the bead race. With a scoop inlet having maximum offset, the inside wall of the inlet is tangent to the outside of the bead race. The airflow from this inlet is directed into the bead chamber via a scoop connection over a longer arc length, in comparison to a tangent inlet where the outer wall of the airway is positioned at a tangent to the bead race, rather than the inner wall of the airway. This entry of air over a longer arc length preserves the driving force to circulate the beads and powder, increasing the exposure of the fluidized powder to the shear stresses created by air flowing rapidly into the chamber from the inlet. Consequently, powder dispersion may be more efficient. This design also reduces the accumulation of particles often seen on the circumference of the bead chamber and immediately before the inlet.

The chord inlet mentioned above extends along a chord, rather than a tangent, to provide different flow and circulation patterns within the bead chamber. Testing shows that powder is dispersed efficiently. The transient accumulation of fluidized particle concentrations at the inside edge of the inlet is reduced and additional airflow shear is present in the area of the bead chamber outside the chord formed by the inlet, which may improve particle dispersion. There also appears to be more and larger scale turbulence within the bead chamber. This may subject particles to greater and more varying shear stresses, which can also enhance dispersion, even while the beads continue to contact all surfaces around the race. This inhaler design has been shown to aerosolize powder efficiently at least as well as tangential inlet design, (similar emitted and respirable doses, 96% versus 92% dose delivery within 0.5 seconds at 30 Ipm, respectively).

The inlets may also have alternate cross sections, smooth or rough walls, and may also include flow directors, to control the flow resistance and flow pattern entering the dispersion chamber. Other shapes and features may be added such as guides at the inlet/bead chamber interface, to retain beads within the chamber, flow directors to control air flow patterns within the bead chamber, or combinations of them and help control air flow patterns. A guide is a bridge or section extending across the inlet opening. A flow director is a structure in front of, or part of, a guide, used to direct flow, such as a vane or louver.

An inhaler according to the invention is assembled by filling the unit dose of medicament into the medicament chamber and then applying the seal member to the surface of the first body member so as to seal the medicament chamber. The first and second body members are then brought together to form the inhaler body, in preferred embodiments by folding about the living hinge or pre-formed fold line. The seal member is sandwiched between the first and second body members, with its free end extending from the inhaler body.

In use, the inhaler may be held substantially horizontally with the first body member uppermost. The user removes the mouthpiece cover if present. The user grasps the end of the seal member and pulls it away from the inhaler body, thereby peeling the seal member away from the medicament chamber and releasing the medicament into the medicament collection well. The user then places the mouthpiece to his or her lips and inhales. The powdered medicament is entrained and dispersed in the airflow passing through the device and passes into the user's respiratory tract.

In an alternative method of use, the inhaler may be held substantially vertically with the first body member facing the user. The user grasps the end of the seal member and pulls it away from the inhaler body, thereby peeling the seal member away from the medicament chamber and releasing the medicament into the combined medicament chamber and collection well, and aiding the presentation by gravity of the powdered medicament to the dispersion chamber.

The first body member, and preferably the whole of the inhaler body, is preferably made from a material that is impermeable to moisture. Such a material may be a plastics material, in which case the first and/or second body members may be manufactured by injection moulding. More preferably, however, the first body member, and preferably also the second body member, are made of metal, and in particular of aluminium or of steel. The seal member may be made of plastics material or metal. Preferred seal members are made from aluminium, and seal members may be laminated or coated with a plastics material to improve the seal and/or facilitate removal. Preferably many, if not all, of the components, including those of the dispersion chamber are made of recyclable materials. This inhaler design is simple, compact, inexpensive, environmentally sensitive, and yet efficient.

The simplicity of design of the inhaler according to the invention renders it possible for it to be manufactured relatively easily in metal, rather than in the plastics material that is more conventional for such devices. The use of metal renders the device particularly resistant to ingress of moisture into the medicament chamber. According to another aspect of the invention, there is therefore provided a unit dose inhaler comprising a first body member and a second body member, the first body member and the second body member being formed in metal and fitting closely together to form an inhaler body, and an outlet, the first body member including a medicament chamber containing a unit dose of powdered medicament and the first body member having affixed to it a sealing mechanism or foil that closes the medicament chamber and which extends outwardly of the inhaler body such that, in use, it can be grasped by a user and withdrawn from the inhaler body, thereby releasing the powdered medicament from the medicament chamber, and the inhaler body including an airway or air flow path communicating with the outlet.

The medicament chamber may hold a powder formulation containing smaller active pharmaceutical particles, and optionally also containing larger inert carrier particles. According to a further aspect of the invention the inhaler includes a dispersion or bead chamber which includes a bead race; and a nosepiece or mouthpiece which has at least one outlet opening connecting or entering into the dispersion chamber. One or more inlets connect into the dispersion chamber from the medicament chamber and/or medicament collection well, adjacent to or in close proximity to the dispersion chamber. The dispersion chamber contains one or more beads which can move about in a bead race. While preferred, the beads are not an essential element. An airway (air flow path or channel) extends past and/or through and/or under the medicament chamber and into the dispersion chamber. A pharmaceutical dry powder formulation is released from the medicament chamber and entrained in air flow through the inhaler when the user inhales on the mouthpiece. The powder is dispersed in air within the dispersion chamber and forms an aerosol inhaled by the user.

Inhalers according to the invention may be used for the delivery of medicaments by inhalation. Thus, according to a further aspect of the invention, there is provided a method of delivering a medicament to a patient, which method comprises the inhalation of the medicament from an inhaler as described above.

A further aspect of the invention relates to the use of a desiccant or desiccants in a dry powder inhaler to improve, e.g. flow and dispersion of said powder. The additive desiccant material preferably consists of a physiologically acceptable hygroscopic salt. It is preferable for only small amounts of additive material to reach the lower lung, and it is also highly preferable for the additive material to be a material which may be safely inhaled into the lower lung where it may be absorbed into the blood stream. The additives are particularly suited for use with hygroscopic and other moisture sensitive agents, e.g. those prone to hydrolysis.

The bioactive agent and desiccant may be co-processed to form a composite. For example, spray-dried pharmaceuticals such as pure insulin may be improved by the inclusion of low levels of said desiccant, preferably sodium sulphate or calcium lactate. Preferred drug:salt ratios are from 0.1:99.9 to 99.9:0.1 % by weight.

The salt may be added to a drug-carrier blend to form a ternary blend for use in a dry powder inhalation (DPI). Here, the desiccant will act as a replacement for lactose fines and there may be a synergistic effect of not only occupying the high energy lactose sites but by imparting moisture resistance to the blend simply absorb moisture from the environment to prevent agglomeration. It is preferred that the desiccant loading in the powder should be not more than 50%, 20 %, 10%, and most preferably not more than 5% based on the weight of the powder.

The carrier particle, e.g. lactose may be substituted with a powdered desiccant exhibiting a similar size distribution and rugosity to conventional DPI lactose, thus acting as a DPI carrier. It is preferred that the desiccant particles have a diameter which lies between 20µm and 1000µm, more preferably between 50µm and 1000µm. Preferably, the diameter of substantially all (by weight) of the desiccant particles is less than 300µm, and more preferably lies between 20µm and 250µm. Preferably at least 90% by weight of the desiccant particles have a diameter between from 50µm to 120µm.

Suitable desiccants include salts such as, but not limited to, ammonium chloride, ammonium orthophosphate, ammonium sulfate, barium chloride dihydrate, calcium lactate pentahydrate, copper sulfate pentahydrate, magnesium salicylate tetrahydrate, magnesium sulfate heptahydrate, potassium bisulfate, potassium bromide, potassium chromate, potassium dihydrogen orthophosphate, sodium acetate trihydrate, sodium bromoiridate dodecahydrate, sodium carbonate decahydrate, sodium fluoride, sodium hydrogen orthophosphate dodecahydrate, sodium metaperiodate trihydrate, sodium metaphosphate trihydrate, sodium hexahydrate, sodium sulfite heptahydrate, sodium sulfate heptahydrate, sodium sulfate decahydrate, sodium thiosulfate pentahydrate, zinc sulfate heptahydrate and combinations thereof

An effective amount of a desiccant salt of this embodiment is one which sufficiently reduces wetting to prevent substantial clumping. Sodium sulfate and calcium lactate are preferred salts.

In the case of the desiccant being a ternary additive or carrier, it may be present in the amorphous, partially-crystalline or crystalline state, having a particle size of preferably less than 20 microns, more preferably less than 10 microns and most preferably less than 5 microns. It may be produced in this state by crystallisation, supercritical processing (e.g. SEDS, GAS, bubble drying, etc), spray-drying, micronization, or any other method known in the art.. The residual moisture content of the salt is preferably between 0 % (totally anhydrous) to 50 % (fully hydrated). In the case of calcium lactate, the preferred moisture content is between 2 and 25 % by weight, more preferably between 10 and 20 % by weight.

A variety of medicaments may be administered by using inhalers of the invention. Such medicaments include those that are suitable for the treatment of asthma, COPD and respiratory infections. Such medicaments include, but are not limited to, β₂-agonists, eg fenoterol, formoterol, pirbuterol, reproterol, rimiterol, salbutamol, salmeterol and terbutaline; non-selective β-stimulants such as isoprenaline; xanthine bronchodilators, eg theophylline, aminophylline and choline theophyllinate; anticholinergics, eg ipratropium bromide, oxitropium and tiotropium; mast cell stabilisers, eg sodium cromoglycate and ketotifen; bronchial anti-infilammatory agents, eg nedocromil sodium; steroids, eg beclomethasone dipropionate, fluticasone, budesonide, flunisolide, triamcinolone, mometasone and ciclesonide, and salts or derivatives thereof; antivirals, eg zanamivir, ribavirin, flumist, ruprintrivir and pleconaril; antibiotics, eg tobramycin, doripenem, pentamidine, promixin, aztreonam; and antifungals, eg amphotericin B (further examples of medicaments that may potentially be administered using inhalers according to the invention are given in Respiratory Care 2000; 45(7): 836-845).

Inhalers according to the invention may also be used to deliver combinations of two or more different medicaments. Specific combinations of medicaments which may be mentioned include combinations of steroids and β₂-agonists. Examples of such combinations are beclomethasone and formoterol; beclomethasone and salmeterol; fluticasone and formoterol; fluticasone and salmeterol; budesonide and formoterol; budesonide and salmeterol; flunisolide and formoterol; flunisolide and salmeterol; ciclesonide and salmeterol; ciclesonide and formoterol; mometasone and salmeterol; and mometasone and formoterol.

In a further aspect of the present invention, the aerosol comprises particles comprising a triptan for the treatment or prophylaxis of migraines. A review of the available literature on the triptan class of compounds show that those with a long duration of action often have a slow onset of action. The opposite is true of compounds that have a fast onset of action. Hence there is an urgent need for fast acting presentations of such drugs or dosage forms with a long duration of action for use in migraine therapy and prophylaxis.

Preferred triptans include, but are not limited to, almotriptan, rizatriptan, naratriptan, zolmitriptan, sumatriptan, eletriptan, frovatriptan, and combinations thereof. Frovatriptan is most preferred. Frovatriptan is a potent 5-HT receptor agonist and has the highest 5-HT potency in the triptan class. Preclinical pharmacology studies demonstrated that frovatriptan is apparently cerebro-selective. In clinical pharmacology studies, frovatriptan was shown to have a long-terminal elimination half-life of 26 h and to be well-tolerated across a broad dose range of 1 to 100 mg. Frovatriptan has no inhibiting or inducing effects on cytochrome P450 isoenzymes and is only slightly bound to plasma proteins; thus it has a low potential for drug interactions. No dosage adjustments are necessary based on age, or renal or hepatic impairment. Efficacy studies show significantly higher response rates compared with placebo and the lowest reported range of headache recurrence rates in the triptan class. Safety studies show a side effect profile similar to placebo. Frovatriptan is particularly well suited to patients with migraine of long duration, those prone to recurrence and those troubled by 'triptan type' side effects.

It has been suggested that as many as 50% of female migraineurs report that menstruation is a trigger for their migraine attacks. Interestingly, menstrually associated migraines often last longer, are generally more resistant to treatment and are more likely to recur. The triptans may be used according to this invention for the prophylaxis and treatment of such menstrually-associated migraines, and delivered as single doses form the disposable inhaler of the invention.

Preferably, the aerosol of the present invention comprises particles comprising at least 5 percent by weight of almotriptan, rizatriptan, naratriptan, zolmikriptan, sumatritpan, eletriptan and preferably frovatriptan. Preferably, the particles comprise at least 10 percent by weight of almotriptan, rizatriptan, naratriptan, zolmitriptan, sumatritpan, eletriptan and preferably frovatriptan. More preferably, the particles comprise at least 20 percent, 30 percent, 40 percent, 50 percent, 60 percent, 70 percent, 80 percent, 90 percent, 95 percent, 97 percent, 99 percent, 99.5 percent or 99.97 percent by weight of almotriptan, rizatriptan, naratriptan, zohnitriptan, sumatritpan, eletriptan and preferably frovatriptan.

Typically, the aerosol has a mass of at least 10 µg. Preferably, the aerosol has a mass of at least 100 µg. More preferably, the aerosol has a mass of at least 200 µg. Typically, the particles are substantially dry, and comprise less than 20 percent, 10 percent, or 5 percent by weight of water.

Typically, the microparticles comprise less than 90 percent by weight of a pharmaceutically acceptable excipient. Preferably, the aerosol comprises less than 80 percent by weight of a pharmaceutically acceptable excipient. More preferably, the dose form comprises less than 70 percent, 60 percent, 50 percent, 40 percent, 30 percent, 20 percent, or 10 percent by weight of a pharmaceutically acceptable excipient.

Such excipients (or fillers or carriers) include sugars such as sucrose, dextrose, lactose, galactose, fructose, trehalose, mixtures thereof, as well as sugar alcohols such as mannitol, sorbitol, xylitol, lactitol, maltitol and galactitol It is preferred that a soluble pharmaceutical filler such as lactose, dextrose, galactose, sucrose, or mixtures thereof be used. In addition, it is to be understood that the above-mentioned sugars and sugar alcohols can also be used as carriers as well, in place of or in addition to the materials described above.

Microparticles comprising the aerosol according to the invention may be prepared (processed) using any suitable technique, including spray-drying, vacuum drying, supercritical processing (e.g. SEDS, RESS, PCA), lyophilisation and milling procedures.

The bioactive material can also be dried from a solution of the carrier and the bioactive material to form a glass containing homogeneously distributed bioactive material in solid solution in the carrier glass. These glasses can then be milled and/or micronized to give microparticles of homogeneous defined sizes. Such solid solutions can provide a quick release of the bioactive upon administration and thereby improve absorption and onset of action.

A particularly suitable technique is spray-freeze-drying technology. The process of spray-freeze-drying involves the atomisation of a solution or dispersion of the carrier and/or therapeutic material, and then directing the resulting droplets into a liquified gas, typically liquid nitrogen, or a cryogenic surface. The droplets freeze on contact and may then be dried using a freeze-drying step to remove residual moisture. The resulting microparticles comprise a therapeutic agent dispersed within the carrier. Prior to microparticle formation, the therapeutic agent may be in solution or present as a dispersion of microparticles or nanoparticles (with an optional stabiliser) in the feedstock.

The apparatus and process conditions used to produce the initial droplets will be apparent to the skilled person. Feed concentrations, pump rates, atomisation pressures and nozzle types can all be selected based on conventional process conditions, and then optimised according to feedstock concentration and viscosity.

The size of the microparticles will be determined in part by the atomisation used in the spray-freeze-drying process. The atomisation/spraying stage may make use of a conventional atomisation process, e.g. pressure or two fluid nozzles, or may utilise an ultrasonic atomisation process (Maa et al., Pharmaceutical Research, 1999; 16(2)). The drying process may be carried out using conventional freeze-drying apparatus.

The most preferred method of production of particles of the aerosol is via spray-drying as fully described in, inter alia, WO 92/18164 and WO 96/15814 (describing the currently preferred process), the contents of which are incorporated herein by reference. Microparticles comprising the aerosol may be spray-dried according to the invention but modified by the inclusion of a blowing agent, in the feedstock for spray-drying. The blowing agent is a volatile substance which releases a gas or gases during the spray-drying process. Blowing agents are used in the present invention, to produce hollow microcapsules. Suitable blowing agents include ammonium acetate, ammonium hydroxide, ammonium carbonate, ammonium bicarbonate, acetic acid, formic acid and hydrochloric acid. The pH at which these blowing agents are used may vary; this implies that compounds with pH-dependent solubilities can be spray-dried with the addition of a suitable blowing agent. By way of example, the blowing agent used in the production of albumin microcapsules is ammonium carbonate which releases ammonia, carbon dioxide and water vapour. During spray-drying, these three gases expand in the atomised droplets, causing the droplet to increase in size, to produce larger, thinner-walled microcapsules. Products of the invention may have various characteristics, depending on the conditions of their preparation. For example, their median size is 1 to 20 µm, and their wall thickness is no more than 500 nm, e.g. 10 to 250 nm, more preferably 100 to 150 nm. Their bulk density may be 0.01 to 0.15 g/cm³, more preferably 0.02 to 0.1 g/cm³, but most preferably 0.04 to 0.8 g/cm³.

The aerosol will usually have a mass mean aerodynamic particle diameter size ranging from 0.1 to 40 microns, preferably from 0.1 to 10 microns, even more preferably from 0.1 to 5 microns and most preferably from 2 to 4 microns. This may be measured using an Aerosizer as will be appreciated by the skilled person.

Drying will usually be carried out to achieve a residual moisture content of the microparticles of less than 10% by weight, preferably less than 5% by weight and most preferably less than 3% by weight. Suitable carrier particles are known, and include crystalline lactose particles and mannitol particles, of a diameter size typically in the range of from 30 to 300 microns, more usually 50 to 250 microns.

The aerosol of the invention comprises particles which may be amorphous, or crystalline, or mixtures thereof. Typically, at least 50 percent by weight of the particles are amorphous in form, wherein crystalline forms make up less than 50 percent by weight of the total aerosol weight, regardless of the nature of individual particles. Preferably, at least 75 percent by weight of the particles are amorphous in form. More preferably, at least 90 percent by weight of the particles are amorphous in form.

In another composition aspect of the present invention, a dose form of an antimigraine compound is provided for the treatment of migraine, wherein the dose form comprises less than the typical oral dose of the antimigraine compound. Typically, where the antimigraine compound is sumatriptan, the dose form comprises less than 20 mg of sumatriptan. Preferably, the dose form comprises less than 15 mg of sumatriptan. More preferably, the dose form comprises less than 10 mg or 5 mg of sumatriptan. Typically, where the antimigraine compound is frovatriptan, the dose form comprises less than 2 mg of frovatriptan. Preferably, the dose form comprises less than 1.75 mg of frovatriptan. More preferably, the dose form comprises less than 1.5 mg, 1.25 mg or 1 mg of frovatriptan.

Salt forms of almotriptan, rizatriptan, naratriptan, zolmitriptan, sumatritpan, eletriptan, and frovatriptan are either commercially available or are obtained from the corresponding free base using well known methods in the art. A variety of pharmaceutically acceptable salts are suitable for aerosolization. Such salts include, without limitation, the following: hydrochloric acid, hydrobromic acid, acetic acid, maleic acid, formic acid, succinic and fumaric acid salts.

Sumatriptan, frovatriptan, and naratriptan are given at strengths of 25 mg, 2.5 mg, and 1 mg respectively for the treatment of migraine headaches. As aerosols, 5 mg to 40 mg of sumatriptan, 0.5 mg to 4 mg of frovatriptan, and 0.2 mg to 2 mg naratriptan are generally provided for the same indication. A typical dosage of a sumatriptan, frovatriptan, or naratriptan aerosol is either administered as a single inhalation or as a series of inhalations taken within an hour or less (dosage equals sum of inhaled amounts). Where the drug is administered as a series of inhalations, a different amount may be delivered in each inhalation. The dosage amount of almotriptan, rizatriptan, naratriptan, zolmitriptan, sumatritpan, eletriptan, and frovatriptan in aerosol form is generally no greater than twice the standard dose of the drug given orally.

A particularly preferred class of bioactive agents suitable for delivery from the unit dose disposable inhaler of this invention are immunogens. The same or different antigens may be present in the microparticles. The immunogens may be used in the prophylaxis of any bacterial or viral disease. For example, the immunogen may be for the prevention of meningococcal disease (meningitis, septicaemia, meningoccaemia and pneumonia). In this embodiment, the immunogen may be used to prevent infection of meningococci of any of groups A, B, C, Y, W135, X and Z. Other suitable immunogens for use in the practice of the invention include vaccines against anthrax (protective antigen), plague, small pox, tularaemia, meliodosis, Q fever, botulism, typhus, cholera, yellow fever, brucellosis, encephalitis, ricin, salmonella and staphylococcal Enterotoxin B. Viral particles useful in the preparation of vaccines are known and are applicable to the invention. The invention may be used for the prophylaxis of HIV, HepB, CMV and TB.

Further medicaments which may be mentioned include systemically active materials, such as, proteinaceous compounds and/or macromolecules, for example, hormones and mediators, such as insulin, human growth hormone, leuprolide and alpha-interferon; growth factors, anticoagulants, immunomodulators, cytokines and nucleic acids.

Where the inhaler of the invention is used to deliver just a single medicament, the device may include only one unit dose of the medicament, in which case the device will generally be disposable and will be discarded immediately after use. Alternatively, the device may include a plurality of individual unit doses, each of which is associated with an individual seal member so that the unit doses can be released for inhalation sequentially. Individual seal members may be numbered to show successive doses.

Where the inhaler is for the delivery of a combination of different medicaments, unit doses of those medicaments may be filled into the same medicament chamber which is then sealed with a seal member, which preferably comprises foil. Alternatively, the different medicaments may occupy different medicament chambers which are sealed by a single seal member. In such a case, when the seal member is removed, the different medicaments may fall into a common medicament collection well or into separate such wells. In a further alternative embodiment, the different medicaments may be stored in separate medicament chambers with separate seal members.

### Predictive Example

A feedstock comprising 10 %w/v frovatriptan succinate was fed to an in-house spray-dryer and was spray dried at the following settings: feed rate=2.1 g/min, inlet temp=130°C, outlet temp=66°C, atomisation=2-fluid nozzle, atomisation pressure=2 barg, atomisation air flow rate=21 l/min, drying air pressure=1 barg, drying air flow rate=5 l/sec. A respirable powder of less than 10 microns was produced which could then be administered to a patient in need of rapid relief from a migraine using the unit-dose dry powder inhaler of the invention.

The invention resides as well in subcombinations of the components, features, methods and steps described. While the drawings and written description may disclose features and components in connection with a specific embodiment, the features and components described below may be used, along or in combinations, with any embodiment. The appearance of these features is a matter of design preference from a large array of options. These features may be selected or changed to create any desired ornamental external appearance of the inhaler.

Thus, a novel unit dose dry powder inhaler has been shown and described. Many changes, substitutions and uses of equivalents may of course be made without departing from the spirit and scope of the invention. The invention therefore, should not be limited, except by the following claims and their equivalents.

Preferred aspects of the invention are set out in the following numbered paragraphs of the description:
1. An inhaler comprising a first body member and a second body member, the first body member and the second body member fitting closely together to form an inhaler body, and an outlet, the first body member including a medicament chamber containing a unit dose of powdered medicament and the first body member having affixed to it a foil that closes the medicament chamber and which extends outwardly of the inhaler body such that, in use, it can be grasped by a user and withdrawn, fully or partially, from the inhaler body, thereby releasing the powdered medicament from the medicament chamber, and the second body member including a medicament collection well in which the medicament is collected when released, in use, from the medicament chamber, the medicament collection well forming part of an airway communicating with the outlet such that, in use, air can be drawn by inhalation at the outlet along the airway to entrain medicament in the medicament collection well for inhalation by a user.
2. An inhaler of paragraph 1, wherein the first body member and the second body member are separate components that are fastened together in order to form the inhaler body.
3. An inhaler of paragraph 1, wherein the first body member and the second body member are hingedly connected.
4. An inhaler of paragraph 3, wherein the first body member and the second body member are formed as a unitary component, being connected by a living hinge or pre-formed fold line.
5. An inhaler of any preceding paragraph, wherein the surfaces of the first and second body members that are brought together to form the inhaler body are substantially planar.
6. An inhaler of paragraph 5, wherein the medicament chamber is formed as a depression in the planar surface of the first body member, and the medicament collection well is formed as a depression in the planar surface of the second body member.
7. An inhaler of any preceding paragraph, wherein the airway is formed as a depression in the second body member.
8. An inhaler of any preceding paragraph, wherein the outlet has the form of a tubular passageway.
9. An inhaler of any preceding paragraph, wherein the foil has the form of an elongate strip, one end portion of which is affixed to the surface of the first body member so as to occlude the opening of the medicament chamber.
10. An inhaler of paragraph 9, wherein the foil is folded back on itself, with the free end of the foil extending out of the inhaler body.
11. An inhaler of any preceding paragraph, which is provided with a visual indicator by which, prior to use, a user may verify that the seal between the foil and the first body member is intact.
12. An inhaler of paragraph 11, wherein said visual indicator is an aperture or window through which a part of the sealing foil is visible.
13. An inhaler of any preceding paragraph, wherein the medicament chamber and the medicament collection well are of comparable dimensions and, after the foil has been removed and the medicament released from the medicament chamber, the medicament chamber and the medicament collection well together form a swirl chamber in which a turbulent airflow is set up during inhalation.
14. An inhaler comprising an inhaler body including a swirl chamber, the swirl chamber being divided by a foil into a first compartment and a second compartment, the first compartment being sealed by the foil and containing a unit dose of powdered medicament and the second compartment being in communication with an outlet, the arrangement being such that removal of the foil releases the medicament from the first compartment such that, in use, air can be drawn by inhalation at the outlet to entrain medicament in the swirl chamber for inhalation by a user.
15. An inhaler of any preceding paragraph, wherein the medicament chamber or first compartment is substantially hemispherical in form.
16. An inhaler of any preceding paragraph, wherein the medicament collection well or second compartment is substantially hemispherical in form.
17. An inhaler of paragraph 15 and paragraph 16, wherein the medicament chamber or first compartment and the medicament collection well or second compartment are substantially hemispherical and have equal diameters, so that together they constitute a substantially spherical swirl chamber.
18. An inhaler of any preceding paragraph, wherein the medicament collection well or second compartment communicates with the outlet via a channel formed in the surface of the second body member.
19. An inhaler of paragraph 18, wherein the channel is of reduced cross-sectional area at its junction with the medicament collection well, so as to form a constriction in the airflow from the medicament collection well to the channel.
20. An inhaler of any preceding paragraph, wherein the first body member is made of metal.
21. An inhaler of any preceding paragraph, wherein the second body member is made of metal.
22. An inhaler of any preceding paragraph, wherein the whole of the inhaler body is made from metal.
23. An inhaler comprising a first body member and a second body member, the first body member and the second body member being formed in metal and fitting closely together to form an inhaler body, and an outlet, the first body member including a medicament chamber containing a unit dose of powdered medicament and the first body member having affixed to it a foil that closes the medicament chamber and which extends outwardly of the inhaler body such that, in use, it can be grasped by a user and withdrawn, fully or partially, from the inhaler body, thereby releasing the powdered medicament from the medicament chamber, and the inhaler body including an airway communicating with the outlet such that, in use, air can be drawn by inhalation at the outlet along the airway to entrain medicament released from the medicament chamber for inhalation by a user.
24. An inhaler of any one of paragraphs 20 to 23, wherein the metal is aluminium or steel.
25. An inhaler of any preceding paragraph, wherein the foil is made of aluminium.
26. An inhaler of any preceding paragraph, which contains a single unit dose of a single medicament.
27. An inhaler of any one of paragraphs 1 to 25, which contains a plurality of unit doses of a single medicament.
28. An inhaler of any one of paragraphs 1 to 25, which contains a single unit dose of each of two or more medicaments.
29. An inhaler of paragraph 28, wherein the unit doses of each of the medicaments are contained within the same medicament chamber.
30. An inhaler of paragraph 28, wherein the unit doses of each of the medicaments are contained in separate medicament chambers.
31. An inhaler of any one of paragraphs 1 to 25, which contains a plurality of unit doses of each of two or more medicaments.
32. A method of delivering a medicament to a patient, which method comprises the inhalation of the medicament from an inhaler as claimed in any preceding paragraph.
33. An inhaler comprising a first body member and a second body member, the first body member and the second body member fitting closely together to form an inhaler body, and an outlet, the first body member including a medicament chamber containing a unit dose of powdered medicament and the first body member having affixed to it a seal member that closes the medicament chamber and which extends outwardly of the inhaler body such that, in use, the seal member can be grasped by a user and withdrawn, fully or partially, from the inhaler body, thereby releasing the powdered medicament from the medicament chamber, the inhaler body including a dispersion chamber, and the inhaler body including an airway communicating with the outlet such that, in use, air can be drawn by inhalation at the outlet along the airway to entrain medicament released from the medicament chamber for dispersion in the dispersion chamber for inhalation by a user.
34. An inhaler according to paragraph 33, wherein the second body member includes a medicament collection well in which the medicament is collected when released, in use, from the medicament chamber.
35. An inhaler according to paragraph 34 wherein the medicament collection well forms part of the airway, such that, in use, the air drawn by inhalation at the outlet along the airway entrains the medicament in the medicament collection well.
36. An inhaler according to any of paragraphs 33 to 35, wherein the first body member and the second body member are separate components that are fastened together in order to form the inhaler body.
37. An inhaler according to any of paragraphs 33 to 35, wherein the first body member and the second body member are hingedly connected.
38. An inhaler according to paragraph 37, wherein the first body member and the second body member are formed as a unitary component, being connected by a living hinge or pre-formed fold line.
39. An inhaler according to any of paragraphs 33 to 38, wherein the surfaces of the first and second body members that are brought together to form the inhaler body are substantially planar.
40. An inhaler according to paragraph 39, wherein the medicament chamber is formed as a depression in the planar surface of the first body member.
41. An inhaler according to paragraphs 39 or 40 wherein the medicament collection well is formed as a depression in the planar surface of the second body member.
42. An inhaler according to any of paragraphs 33 to 41, wherein the airway is formed as a depression in the first body member and/or second body member.
43. An inhaler according to any of paragraphs 33 to 42, wherein the outlet has the form of a tubular passageway.
44. An inhaler according to any of paragraphs 33 to 43, wherein the seal member comprises an elongate strip, one end portion of which is affixed to the first body member so as to occlude the opening of the medicament chamber.
45. An inhaler according to paragraph 44, wherein the seal member is folded back on itself, with the free end of the seal member extending out of the inhaler body.
46. An inhaler according to any of paragraphs 33 to 45, which is provided with a visual indicator by which, prior to use, a user may verify that the seal between the seal member and the first body member is intact.
47. An inhaler according to paragraph 46, wherein said visual indicator comprises an aperture or window through which a part of the seal member may be viewed.
48. An inhaler according to any of paragraphs 44 to 47, wherein the medicament chamber and the medicament collection well are of comparable dimensions and, after the seal member has been removed and the medicament released from the medicament chamber, the medicament chamber and the medicament collection well together form a swirl chamber in which a turbulent airflow is set up during inhalation.
49. An inhaler according to any of paragraphs 33 to 48, wherein the medicament chamber is substantially hemispherical in form.
50. An inhaler according to any of paragraphs 34 to 49, wherein the medicament collection well is substantially hemispherical in form.
51. An inhaler according to any of paragraphs 34 to 50, wherein the medicament chamber and the medicament collection well are substantially hemispherical and have substantially equal diameters, so that together they constitute a substantially spherical swirl chamber.
52. An inhaler according to any of paragraphs 32 to 51, wherein the medicament collection well communicates with the outlet via a channel formed in the surface of the first body member and/or second body member.
53. An inhaler according to paragraph 52, wherein the channel is of reduced cross-sectional area at its junction with the medicament collection well, so as to form a constriction in the airway from the medicament collection well to the channel.
54. An inhaler according to any of paragraphs 33 to 53, wherein the first body member is made of metal.
55. An inhaler according to any of paragraphs 33 to 54, wherein the second body member is made of metal.
56. An inhaler according to any of paragraphs 33 to 55, wherein the whole of the inhaler body is made from metal.
57. An inhaler according to any of paragraphs 54 to 56, wherein the metal is aluminium or steel.
58. An inhaler according to any of paragraphs 33 to 57 wherein the seal member comprises foil.
59. Inhaler according to paragraph 58, wherein the foil comprises aluminium.
60. An inhaler according to any of paragraphs 33 to 59, which contains a single unit dose of a single medicament.
61. An inhaler according to any of paragraphs 33 to 59, which contains a plurality of unit doses of a single medicament.
62. An inhaler according to any of paragraphs 33 to 59, which contains a single unit dose of each of two or more medicaments.
63. An inhaler according to paragraph 62, wherein the unit doses of each of the medicaments are contained within the same medicament chamber.
64. An inhaler according to paragraph 62, wherein the unit doses of each of the medicaments are contained in separate medicament chambers.
65. An inhaler according to any of paragraphs 33 to 59, which contains a plurality of unit doses of each of two or more medicaments.
66. An inhaler according to any of paragraphs 33 to 65 wherein the dispersion chamber contains at least one bead, the at least one bead being dimensioned so that it can move around the dispersion chamber.
67. An inhaler according to paragraph 66 wherein the dispersion chamber comprises a peripheral bead race, and wherein the at least one bead can move about in the bead race.
68. An inhaler according to paragraph 66 or 67 wherein the dispersion chamber comprises from 2 to 10 beads.
69. An inhaler according to any of paragraphs 66 to 68 wherein the dispersion chamber comprises a plurality of beads and at least one bead has a shape different from at least one other bead.
70. An inhaler according to any of paragraphs 66 to 69 wherein the dispersion chamber comprises means to cause the at least one bead to move in a chaotic manner.
71. An inhaler according to paragraph 70 wherein the means to cause the at least one bead to move in a chaotic manner comprises an obstruction in the dispersion chamber.
72. An inhaler according to any of paragraphs 33 to 71 further comprising means to generate a sheath of air within the periphery of the outlet when air is drawn by inhalation at the outlet.
73. An inhaler according to paragraph 72 wherein the means for generating the sheath of air comprises a plurality of air inlets in the outlet.
74. An inhaler according to any preceding claim wherein the medicament comprises a triptan.
75. An inhaler according to paragraph 74 wherein the triptan is selected from almotriptan, rizatriptan, naratriptan, zolmitriptan, sumatritpan, eletriptan, frovatriptan, and combinations thereof.
76. An inhaler according to paragraph 74 or 75 wherein the triptan is frovatriptan.
77. A dry powder for inhalation comprising a desiccant and a bioactive agent.
78. A dry powder according to paragraph 77 wherein the bioactive agent is selected from β₂-agonists, fenoterol, formoterol, pirbuterol, reproterol, rimiterol, salbutamol, salmeterol, terbutaline, non-selective β-stimulants, isoprenaline, xanthine bronchodilators, theophylline, aminophylline, choline theophyllinate, anticholinergics, ipratropium bromide, oxitropium, tiotropium, mast cell stabilisers, sodium cromoglycate, ketotifen, bronchial anti-inflammatory agents, nedocromil sodium, steroids, beclomethasone dipropionate, fluticasone, budesonide, flunisolide, triamcinolone, mometasone, ciclesonide, antivirals, zanamivir, ribavirin, fiumist, I-ruprintrivir, pleconaril antibiotics, tobramycin, doripenem, pentamidine, promixin, aztreonam, antifungals, amphotericin B, and combinations thereof.
79. A dry powder according to paragraph 77 wherein the bioactive agent is selected from immunogens for the prevention or treatment of meningococcal disease, meningitis, septicaemia, meningoccaemia, pneumonia, meningococci of any of groups A, B, C, Y, W135, X and/or Z, anthrax, plague, small pox, tularaemia, meliodosis, Q fever, botulism, typhus, cholera, yellow fever, brucellosis, encephalitis, ricin, salmonella, staphylococcal Enterotoxin B, HIV, HepB, CMV, TB, and combinations thereof.
80. A dry powder according to paragraph 77 wherein the bioactive agent is selected from proteinaceous compounds, macromolecules, hormones, mediators, insulin, human growth hormone, leuprolide, alpha-interferon, growth factors, anticoagulants, immunomodulators, cytokines, nucleic acids and combinations thereof.
81. A dry powder composition according to paragraph 77 wherein the bioactive agent is a triptan.
82. A dry powder according to paragraph 81 wherein the triptan comprises almotriptan, rizatriptan, naratriptan, zolmitriptan, sumatritpan, eletriptan, frovatriptan, and combinations thereof.
83. A dry powder according to paragraph 81 or 82 wherein the triptan comprises frovatriptan.
84. A dry powder according to any of paragraphs 77 to 83 further comprising a carrier.
85. A dry powder according to any of paragraphs 77 to 84 which comprises the desiccant and the bioactive agent in a ratio of from 0.1:99.9 to 99.9:0.1 % by weight.
86. A dry powder according to any of paragraphs 77 to 85 which comprises the desiccant in an amount of not more than about 50% by weight, preferably not more than about 20 % by weight, more preferably not more than about 10% by weight, and most preferably not more than about 5% based on the weight of the powder.
87. A dry powder according to any of paragraphs 77 to 86 wherein the desiccant comprises particles exhibiting a similar size distribution and rugosity to conventional DPI lactose.
88. A dry powder according to any of paragraphs 77 to 87 wherein the desiccant comprises particles having a diameter which lies between about 20µm and about 1000µm, more preferably between about 50µm and about 1000µm.
89. A dry powder according to any of paragraphs 77 to 87 wherein the desiccant comprises particles, substantially all of which (by weight) have a diameter of less than about 300µm, and preferably between about 20µm and about 250µm.
90. A dry powder according to any of paragraphs 77 to 87 wherein the desiccant comprises particles, at least 90% by weight of which have a diameter of from between about 50µm and about 120µm.
91. A dry powder according to any of paragraphs 77 to 87 wherein the desiccant comprises ammonium chloride, ammonium orthophosphate, ammonium sulfate, barium chloride dihydrate, calcium lactate pentahydrate, copper sulfate pentahydrate, magnesium salicylate tetrahydrate, magnesium sulfate heptahydrate, potassium bisulfate, potassium bromide, potassium chromate, potassium dihydrogen orthophosphate, sodium acetate trihydrate, sodium bromoiridate dodecahydrate, sodium carbonate decahydrate, sodium fluoride, sodium hydrogen orthophosphate dodecahydrate, sodium metaperiodate trihydrate, sodium metaphosphate trihydrate, sodium metaphosphate hexahydrate, sodium sulfite heptahydrate, sodium sulfate heptahydrate, sodium sulfate decahydrate, sodium thiosulfate pentahydrate, zinc sulfate heptahydrate and combinations thereof.
92. A dry powder according to any of paragraphs 77 to 91 wherein the desiccant comprises sodium sulfate and/or calcium lactate.
93. A method of manufacturing a dry powder as defined in any of paragraphs 77 to 92 comprising mixing the desiccant with the bioactive agent.
94. A method according to paragraph 93 wherein the desiccant is spray dried with the bioactive agent.
95. A method according to paragraph 93 or 94, further comprising the step of adding a unit dose of the dry powder to an inhaler.
96. A method according to paragraph 95 wherein the inhaler is as defined in any of paragraphs 1 to 73.
97. An inhaler containing a dry powder as defined in any of paragraphs 77 to 92.
98. An inhaler according to paragraph 97 wherein the inhaler is as defined in any of paragraphs 1 to 73.
99. A method of delivering a medicament to a patient, which method comprises the inhalation of the medicament from an inhaler as defined in any of paragraphs 1 to 73.
100. A method according to paragraph 99 wherein the medicament comprises a dry powder as defined in any of paragraph 77 to 92.
101. A method of delivering a medicament to a patient, which method comprises the inhalation of a medicament comprising a dry powder as defined in any of paragraph 77 to 92 from an inhaler.
102. A method according to paragraph 101 wherein the inhaler is as defined in any of paragraphs 1 to 73.
103. A method according to any of paragraph 99 to 102 wherein the medicament is for the prevention or treatment of migraines.
104. A method according to any of paragraphs 99 to 103 comprising the step of releasing the medicament from the medicament chamber by withdrawing the foil or seal member, fully or partially, from the inhaler body.
105. An inhaler substantially as described herein and/or as shown in the figures.
106. A dry powder for inhalation substantially as described herein and/or as shown in the figures.
107. A method of manufacturing a dry powder for inhalation substantially as described herein and/or as shown in the figures.
108. A method of delivering medicament to a patient substantially as described herein and/or as shown in the figures.

## Claims

1. A dry powder for inhalation comprising a desiccant and a bioactive agent.

2. A dry powder according to Claim 1 wherein the bioactive agent is selected from β₂-agonists, fenoterol, formoterol, pirbuterol, reproterol, rimiterol, salbutamol, salmeterol, terbutaline, non-selective β-stimulants, isoprenaline, xanthine bronchodilators, theophylline, aminophylline, choline theophyllinate, anticholinergics, ipratropium bromide, oxitropium, tiotropium, mast cell stabilisers, sodium cromoglycate, ketotifen, bronchial anti-inflammatory agents, nedocromil sodium, steroids, beclomethasone dipropionate, fluticasone, budesonide, flunisolide, triamcinolone, mometasone, ciclesonide, antivirals, zanamivir, ribavirin, flumist, ruprintrivir, pleconaril antibiotics, tobramycin, doripenem, pentamidine, promixin, aztreonam, antifungals, amphotericin B, and combinations thereof.

3. A dry powder according to Claim 1 wherein the bioactive agent is selected from immunogens for the prevention or treatment of meningococcal disease, meningitis, septicaemia, meningoccaemia, pneumonia, meningococci of any of groups A, B, C, Y, W135, X and/or Z, anthrax, plague, small pox, tularaemia, meliodosis, Q fever, botulism, typhus, cholera, yellow fever, brucellosis, encephalitis, ricin, salmonella, staphylococcal Enterotoxin B, HIV, HepB, CMV, TB, and combinations thereof.

4. A dry powder according to Claim 1 wherein the bioactive agent is selected from proteinaceous compounds, macromolecules, hormones, mediators, insulin, human growth hormone, leuprolide, alpha-interferon, growth factors, anticoagulants, immunomodulators, cytokines, nucleic acids and combinations thereof; preferably wherein the bioactive agent is a triptan.

5. A dry powder according to Claim 4 wherein the triptan comprises almotriptan, rizatriptan, naratriptan, zolmitriptan, sumatriptan, eletriptan, frovatriptan, and combinations thereof; preferably wherein the triptan comprises frovatriptan.

6. A dry powder according to any of Claims 1 to 5 further comprising a carrier.

7. A dry powder according to any of Claims 1 to 6 which comprises the desiccant and the bioactive agent in a ratio of from 0.1:99.9 to 99.9:0.1 % by weight.

8. A dry powder according to any of Claims 1 to 7 which comprises the desiccant in an amount of not more than about 50% by weight, preferably not more than about 20 % by weight, more preferably not more than about 10% by weight, and most preferably not more than about 5% based on the weight of the powder.

9. A dry powder according to any of Claims 1 to 8 wherein the desiccant comprises particles exhibiting a similar size distribution and rugosity to conventional DPI lactose.

10. A dry powder according to any of Claims 1 to 9 wherein the desiccant comprises particles having a diameter which lies between about 20 µm and about 1000 µm, more preferably between about 50 µm and about 1000 µm; preferably wherein substantially all of which (by weight) have a diameter of less than about 300 µm, and preferably between about 20 µm and about 250 µm.

11. A dry powder according to any of Claims 1 to 9 wherein the desiccant comprises particles, at least 90% by weight of which have a diameter of from between about 50 µm and about 120 µm.

12. A dry powder according to any of Claims 1 to 9 wherein the desiccant comprises ammonium chloride, ammonium orthophosphate, ammonium sulfate, barium chloride dihydrate, calcium lactate pentahydrate, copper sulfate pentahydrate, magnesium salicylate tetrahydrate, magnesium sulfate heptahydrate, potassium bisulfate, potassium bromide, potassium chromate, potassium dihydrogen orthophosphate, sodium acetate trihydrate, sodium bromoiridate dodecahydrate, sodium carbonate decahydrate, sodium fluoride, sodium hydrogen orthophosphate dodecahydrate, sodium metaperiodate trihydrate, sodium metaphosphate trihydrate, sodium metaphosphate hexahydrate, sodium sulfite heptahydrate, sodium sulfate heptahydrate, sodium sulfate decahydrate, sodium thiosulfate pentahydrate, zinc sulfate heptahydrate and combinations thereof.

13. A dry powder according to any of Claims 1 to 12 wherein the desiccant comprises sodium sulfate and/or calcium lactate.

14. A method of manufacturing a dry powder as defined in any of Claim 1 to 13 comprising mixing the desiccant with the bioactive agent; preferably wherein the desiccant is spray dried with the bioactive agent; optionally further comprising the step of adding a unit dose of the dry powder to an inhaler.

15. An inhaler containing a dry powder as defined in any of Claims 1 to 14.
